Europäisches Patentamt

European Patent Office

Office européen des brevets

(11) Publication number: **0 198 490 B1**

## EUROPEAN PATENT SPECIFICATION

(45) Date of publication of patent specification: **27.05.92**

(51) Int. Cl.⁵: **A61K 9/08**, G02C 13/00, G02C 7/04

(21) Application number: **86105269.4**

(22) Date of filing: **16.04.86**

(54) **Ophthalmic solutions and methods for improving the comfort and safety of contact lenses.**

(30) Priority: **19.04.85 US 725065**

(43) Date of publication of application:
**22.10.86 Bulletin 86/43**

(45) Publication of the grant of the patent:
**27.05.92 Bulletin 92/22**

(84) Designated Contracting States:
**AT BE CH DE FR GB IT LI LU NL SE**

(56) References cited:
**EP-A- 0 055 048     EP-A- 0 076 136**
**EP-A- 0 079 185     FR-A- 2 515 201**
**US-A- 3 689 673     US-A- 4 323 467**
**US-A- 4 356 100     US-A- 4 409 205**
**US-A- 4 510 065**

(73) Proprietor: **CIBA-GEIGY AG**
**Klybeckstrasse 141**
**CH-4002 Basel(CH)**

(72) Inventor: **Su, Kai Chiang**
**13170 Hopewell Road**
**Alpharetta, GA 30201(US)**
Inventor: **Winterton, Lynn**
**1445 Parkmont Drive**
**Roswell, GA 30076(US)**

(74) Representative: **Zumstein, Fritz, Dr. et al**
**Bräuhausstrasse 4**
**W-8000 München 2(DE)**

EP 0 198 490 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention is directed to ophthalmic solutions for improving the safety and comfort of wearers of contact lenses and to methods for improving the comfort and safety of wearers of such lenses by means of such solutions.

Contact lenses have provided a useful alternative to eyeglasses for correcting faulty vision. Originally, contact lenses were made of a hard plastic such as methyl methacrylate polymers. More recently, soft contact lenses more comfortable than the hard lenses have become available. Soft contact lenses may be produced by using hydrogels such as those comprising polymers of hydroxyethyl methacrylate.

Hydrogel contact lenses can be arbitrarily divided into those suitable only for daily wear (daily wear lenses) and those suitable for extended wear in addition to daily wear (extended wear lenses). Daily wear lenses are removed every day for cleaning. Extended wear lenses may be worn more than one day before they are removed for cleaning. For example, extended wear lenses may be worn up to about 9 months and even more before removal.

Hydrogel lenses suitable for extended wear generally have higher water contents and/or are thinner than those suitable for daily wear. For example, hydrogel extended wear lenses typically have water contents of at least about 42 %, preferably at least about 45 % and most preferably at least about 55 % by weight and/or a center thickness less than about 0.045 mm, preferably less than about 0.040 mm, and most preferably less than about 0.035 mm. Hydrogel contact lenses suitable for daily wear typically have a water content less than about 55 %, preferably less than 45 % and/or a center thickness of at least about 0.05 mm, preferably at least about 0.06 mm.

The designation of contact lenses as suitable for daily wear or for extended wear depends on performance and not on its compositions or structure. In order to obtain approval by the United States Food and Drug Administration as suitable for extended wear, a lens must be able to be slept in safely. According to an article by Polse and Decker in Invest. Ophthal. Visual Sci. 18. 188 (1979), a contact lens should have an oxygen transmissibility of $5 \times 10^{-9}$ and $15 \times 10^{-9}$ cm x ml $O_2$/s x $\overline{\text{ml}}$ x $10^5$/760 Pa (Cm * ml $O_2$/s * ml * mm Hg) for open and closed eyes, respectively, in order to provide the necessary oxygen flux of 2 l/cm² hr to the cornea.

In the present specification and claims, a hydrogel contact lens having a center thickness less than about 0.045 mm, preferably less than about 0.040 mm and most preferably less than about 0.035 mm will be referred to as an "ultrathin" lens. A hydrogel contact lens having a water content of at least about 42 %, preferably at least about 45 %, and most preferably about 55 % will be referred to as a "high water content" lens.

The comfort of a contact lens in an eye depends in part on the exchange of fluid in the area outside the lens with that in the area underneath the lens (i.e. between the lens and the eye). Such exchange will be referred to as "tear exchange" in the discussion below.

Hard contact lenses undergo a pumping action due to the pressure exerted by the upper eye lid on the top of the lens, which acts as a fulcrum. This pumping causes the desired exchange of fluid.

Such "pumping", however, does not occur with soft contact lenses; see Mandel, "Contact Lens Practice" 3rd ed, 1981, page 512. Accordingly, there is reduced tear exchange in the eyes of wearers of soft contact lenses. This reduced tear exchange is disadvantageous because it prevents the removal of waste matter and debris from the area underneath the lens. This waste matter and debris, which includes proteins, cells, metabolic products, fragments thereof, and the like, become trapped under the lens. The accumulation of the trapped waste matter and debris contribute to the discomfort of the wearer, and in extreme cases may be harmful to the eye.

At the same time, desirable components of the tear fluid outside the contact lens are prevented from contacting the portion of the eye underneath the lens. Such components may occur naturally in the eye, such as oxygen, or artificially, such as antibiotics or other pharmaceutically active compounds.

Although the efficient pumping action of the hard contact lenses does not occur with soft contact lenses, there is still some movement of a soft contact lens in an eye due to the pressure of the upper eye lid on the lens. This movement permits some tear exchange to occur, but is not always sufficient to prevent the accumulation of waste matter and debris under the lens. Moreover, this desirable movement of the lens in the eye tends to decrease with time as the lens dehydrates, shrinks, and grips the eye more tightly.

Isotonic solutions for improving the comfort of wearing soft contact lenses are known. Such solutions typically contain viscosity enhancing agents, lubricants, surfactants, buffers, preservatives, and salts, and are added directly to a contact lens-containing eye. An example of such a solution is Clerz, manufactured and marketed by Coopervision. The Clerz formula contains, by weight, 0.1 % sorbic acid, 0.1 % disodium edetate, 0.22 % sodium borate, and 1 % poloxamer 407, which is a block copolymer of ethylene oxide and propylene oxide.

Sherman discloses in US-A-4,356,100 an aqueous composition for cleaning rinsing and cold disinfecting of soft contact lenses during non-wearing periods. Said compositions comprise, as an essential ingredient, 0.2 % to 5 % propylene glycol.

European patent application 079,185 is directed to isotonic cleaning solutions for contact lenses based on a low dosis of chlorhexidene salt. Cleaning is effected e.g. by rubbing a lens between palm and forefinger of the right hand. Such solutions are made isotonic with for example glycerol, propylene glycol or urea. Amounts of 2.0 % or 2.5 % of these non-ionic tonicity agents are disclosed.

Phares discloses in US-A-3,689,673 chlorhexidene solutions for sterilizing and soaking contact lenses. These solutions are preferably made isotonic by sodium chloride or materials such as propylene glycol or glycerine. Amounts of 2.0 % of these tonicity agents are disclosed, and the solutions are provided for use during non-wearing periods.

Fu discloses in US-A-4,323,467 a solution for cleaning, storing or wetting contact lenses when the lens is not worn, which are preferably isotonic. Tonicity is adjusted by adding 0.4 % to 1.7 % of a tonicity agent. The preferred tonicity agent is sodium chloride, but even glycerol is mentioned. However, it is known in the art that glycerol levels as low as 0.4 % or 0.5 % are unsuitable for rendering a solution isotonic. Thus, in view of the purpose for which glycerol is mentioned by Fu, concentrations of up to and including 0.5 % of glycerol are neither taught nor suggested by Fu.

Sherman discloses in US-A-4,510,065 preservative systems for the daily cleaning of contact lenses which may comprise 0.005 % to 5 % propylene glycol. Again, there is no hint that these solutions may be used other than with a lens which is not worn in the eye.

Shively discloses solutions for use by contact lens wearers who have irregularly structured tear films; see Shively U.S. patent 4,409,205. Such solutions should be hypotonic; see Shively, "Development of Clinically Acceptable Artificial Tear Formulations" in Symposium: Ophthalmic Drug Delivery Systems, Joseph R. Robinson, ed., Am. Pharm. Assoc., publishers, Kansas City, MO, 1980. The solutions of the present invention, are, by comparison, isotonic and, therefore, especially beneficial for contact lens wearers having regularly structured tear films.

The known solutions for improving contact lens comfort have not been satisfactory in improving tear exchange. In fact, some of the known solutions prevent such exchange by causing the lens to grip the eye more tightly than the lens would in the absence of the solution. This tighter grip of the eye by the lens decreases the movement of the lens in the eye, and prevents the desired tear exchange.

Accordingly, a need exists for ophthalmic solutions which will loosen the grip of soft, hydrogel contact lenses on the eye and/or will increase the movement of the lens in the eye, permitting an increase in tear exchange.

The principal object of the present invention is to provide ophthalmic solutions which increase the tear exchange in human eyes containing soft, hydrogel contact lenses, contributing to the comfort and safety of wearing such lenses.

In the following description and claims of the present invention, percent is by weight unless otherwise indicated.

The objects of the present invention have been obtained by providing an ophthalmic solution for improving the exchange of fluid in the area outside a hydrogel contact lens with the fluid in the area underneath the hydrogel contact lens comprising:

for a daily wear hydrogel contact lens,

a) 0.1 to 0.5 % by weight of a lens flattening agent selected from urea, glycerin, sorbitol, aminoethanol and mixtures thereof; provided that the amount of glycerin is below 0.4 % by weight;

b) an ocularly acceptable ionic salt in an amount sufficient to give the entire comfort drop solution a tonicity in the range of 270 - 330 milliosmoles per kilogram of said comfort drop solution;

c) an ocularly acceptable viscosity enhancing agent in an amount sufficient to give said comfort drop solution a Brookfield relative viscosity in the range of 3 to 50 mPa•s (cps);

d) an ocularly acceptable buffer in an amount of 0 % or an effective buffering amount to result in said comfort drop solution having a pH of 6.5 to 8;

e) 0 % to 0.5 % of an ocularly acceptable preservative;

f) 0 % to 2 % of an ocularly acceptable lubricant;

g) 0 % or an ocularly acceptable pharmaceutically effective amount of an ocularly administrable pharmaceutically active agent; and

h) 0 % to 1 % of a non-ionic ocularly acceptable surfactant;

or for an extended wear hydrogel contact lens

a) 0.0005 to 0.1 % by weight of a lens flattening agent selected from urea, glycerin, sorbitol, aminoethanol and mixtures thereof;

b) an ocularly acceptable ionic salt in an amount sufficient to give the entire comfort drop solution a tonicity in the range of 270 - 330 milliosmoles per kilogram of said comfort drop solution;

c) an ocularly acceptable viscosity enhancing agent in an amount sufficient to give said comfort drop solution a Brookfield relative viscosity in the range of 3 to 50 mPa•s (cps);

d) an ocularly acceptable buffer in an amount of 0 % or an effective buffering amount to result in said comfort drop solution having a pH of 6.5 to 8;

e) 0 % to 0.5 % of an ocularly acceptable preservative;

f) 0 % to 2 % of an ocularly acceptable lubricant;

g) 0 % or an ocularly acceptable pharmaceutically effective amount of an ocularly administrable pharmaceutically active agent; and

h) 0 % to 1 % of a non-ionic ocularly acceptable surfactant.

The invention also includes a method for improving the exchange of fluid in the area outside a hydrogel contact lens with the fluid in the area underneath the hydrogel contact lens in a human eye comprising adding to said eye an effective amount of such ophthalmic solutions.

The gist of the present invention is the discovery that certain compounds cause the base curve of soft, hydrogel contact lenses to flatten significantly. The compounds which cause the flattening of hydrogel contact lenses are known to cause swelling of hydrogels.

This swelling is ordinarily expected to be uniform along the base curve diameter and the sagital height of the contact lens. Such uniform swelling would not flatten the base curve of the lens and would not be expected to significantly cause the lens to move more freely on the cornea of the eye.

It has now been found, however, that some compounds unexpectedly cause swelling preferentially along the base curve diameter of the lens relative to the sagital height. Such preferential swelling causes a temporary flattening of the base curve, inducing a loosening of the grip of the eye by the lens and greater movement of the lens in the eye. This loosening and increased movement, in turn, causes the increased tear exchange. Movement of the lens in the eye double and even greater than double the normal movement may be achieved by means of the present solutions.

The contact lens flattening agent is eventually washed away by the natural fluids of the eye, causing the contact lens to return to its original shape. During the time the base curve is distorted, vision is not normally significantly hampered. Comfort and safety are improved by the flushing of waste matter and debris from underneath the lens. At the same time, desirable components from the eye fluid outside the area under the lens, such as oxygen and pharmaceutically active compounds come into contact with the area of the eye underneath the lens.

Extended wear contact lenses are maintained in the eye for longer periods of time than are daily wear lenses. Accordingly, the solutions of the present invention are especially useful for wearers of extended wear contact lenses.

Any hydrogel swelling agent which swells a hydrogel preferentially along the base curve diameter preferentially relative to the sagital height and which is suitable for use in a human eye may be used in the present solutions and methods as the contact lens flattening agent. Suitable contact lens flattening agents include, for example, urea, glycerin, sorbitol, (both 1- and 2-)amino ethanol and mixtures of these agents. The preferred flattening agent is urea.

The concentration of the contact lens flattening agent may be any concentration that will cause the desired effect and still provide an isotonic or substantially isotonic solution. The optimum concentration will depend on various factors, such as the nature of the contact lens and the particular flattening agent used. The concentration will normally be at least 0.0005 % by weight, preferably at least 0.005 % by weight and most preferably at least 0.01 % by weight. In any solution having less than a substantially isotonic amount of the flattening agent, the other ingredients are used as set forth below, so that the final product is substantially isotonic.

The maximum concentration of the contact lens flattening agent is that amount equivalent to 330, preferably 320, and most preferably 310 milliosmoles/kg.

It has been found that concentrations of contact lens flattening agents greater than about 1 % by weight cause a delay in the desired effect until the agent is diluted by the normal functioning of the eye. Accordingly, the maximum concentration is preferably less than 1 %, more preferably less than about 0.5 % and most preferably less than about 0.3 % by weight.

The optimal concentration of the flattening agents useful with the ultrathin and/or high water content lenses as defined above, generally suitable for extended wear is different from the optimal concentration useful with thicker, lower water containing lenses suitable for daily wear. The optimal concentration of the flattening agent useful with ultrathin and/or high water content lenses is 0.0005 to 0.1 %, preferably 0.001 to

EP 0 198 490 B1

0.08 %, and most preferably 0.01 to 0.05 % by weight.

The optimal concentration of the flattening agent useful with daily wear contact lenses is 0.1 to 0.3 % by weight.

The solutions useful in the present invention are substantially isotonic. Substantially isotonic solutions are defined for present purposes as containing 270-330, preferably 280-320, and most preferably 290-310 milliosmoles/kg of solutes. The tonicity adjusting agent is employed to bring the final solution tonicity within the stated ranges if not already there due to contributions of the other ingredients.

The preferred isotonicity adjusting agents are ionic salts, e.g. alkali metal halides, especially sodium chloride. The solutions of the invention preferably contain an ionic salt in an amount equivalent to at least 0.3 % sodium chloride, preferably at least 0.5 % and most preferably at least 0.75 %. When determining the concentration of the tonicity adjusting agent, the contribution to tonicity of the contact lens flattening agent and other ingredients present must be taken into account.

The Brookfield relative viscosity of the present invention is preferably adjusted to 3 to 50, preferably 5 to 20, and most preferably 6 to 11 mPa·s (cps) by means of a viscosity enhancing agent. Some suitable viscosity enhancing agents include, for example, cellulosic polymers such as hydroxyethyl cellulose, methyl cellulose and carboxymethyl cellulose; polymeric polyalkylene oxides such as polypropylene oxide and polyethylene oxide; polyoxypropylene-polyoxyethylene block copolymers; and polyvinyl alcohol. Viscosities less than 3 mPa·s (cps) are possible, but there may be a delay in the flattening effect after the solutions are placed in the eye.

The viscosity enhancing agent should not coat the lens. This is a particular problem with higher molecular weight viscosity enhancing agents. A particularly suitable viscosity enhancing agent is the hydroxyethyl cellulose sold by Union Carbide as QP40, of which a 2 % solution has a Brookfield relative viscosity of 80 to 125 mPa·s (cps) when measured with a number 1 spindle at 30 revolutions per minute.

The hydrogel contact lenses suitable for use with the present solutions and methods are the usual "soft" contact lenses, which are already well known and which have been popularly accepted by consumers. These lenses are generally made from e.g. polymeric hydroxyethyl methacrylate, ethylene glycol dimethacrylate, vinylpyrrolidone, and mixtures thereof. Other hydrogel materials are also known.

Optional ingredients such as preservatives, buffers, surfactants, lubricants, pharmaceutically active compounds and vasoconstricting (i.e. decongesting) agents may also be included in the solutions of this invention.

Preservatives useful in the present invention should not cause irritation to the eye. Strong binding of the preservative to the lens is also undesirable since it causes the preservative to accumulate in the eye. Accordingly, preservatives such as chlorhexidine and those containing quaternary ammonium compounds, such as benzalkonium chloride and mercury-containing organocompounds, such as thimerosal sodium and phenylmercuric acetate are disadvantageous and preferably excluded from the solutions.

Preservatives preferred for use in the present solutions are any effective, non-irritating preservative which is compatible with hydrogels. Some suitable preservatives include sorbic acid and EDTA. The preferred preservative is sorbic acid.

Any biostatic amount of preservative which prevents contamination of the solutions may be used. Some suitable preservative concentrations include 0.001 to 0.5 %, preferably 0.1 to 0.25 %.

The solutions of the present invention are preferably buffered at pH 6.5 to 8, and preferably at pH 7 to 7.6, more preferably at pH 7.3 to 7.4, most preferably at pH 7.3. Some suitable buffers include borate, acetate, citrate, and phosphate buffer systems.

The borate buffers are preferred since it has been unexpectedly found that the contact lens movement promoting agents, and especially urea, are more effective in the presence of the borate buffers. The buffer system is used at a concentration which provides the desired pH. The combined concentration of boric acid and sodium tetraborate decahydrate, the preferred borate buffer components, is, in the completed solution of the invention, typically from 0.2 to 0.6 %.

The surfactants useful in the present invention are non-irritating to the eye and are, preferably, non-ionic. Some suitable examples of surfactants include ethylene oxide-propylene oxide block copolymers such as members of the poloxamer series including Poloxamer 407, Poloxamer 338 and Poloxamer 288. Preferred is Poloxamer 407 sold by BASF Wyndotte under the name Pluronic 127.

Other suitable surfactants are polyethylene oxides such as WSRN 10, WSRN 12K and WSRN 60K sold by Union Carbide.

A third suitable surfactant is Tyloxapol, which is an oxyethylated tertiary octylphenol formaldehyde polymer.

The amount of the surfactant may be, for example, about 0.01 to 1 % and preferably 0.1 to 0.5 %.

Some suitable lubricants include, for example, hydroxyethylcellulose, polyvinyl alcohol and polyvinylpyrrolidone.

The lubricants may be present in amounts of 0.01 to 2 % and preferably 0.1 to 1 %.

The pharmaceutically active compounds useful in the solutions of this invention are those which have a prophylactic or therapeutic effect on eye disorders. Some examples of pharmaceutically active compounds include, for example, compounds for the treatment of glaucoma, e.g. pilocarpine hydrochloride, compounds for the treatment of red eyes, e.g. phenylephrin, and compounds for inflammatory ocular conditions, e.g. dexamethasone or fluorometholone. Various anti-microbial pharmaceuticals for treatment of diseases of mucous membranes may be used, such as clofazimine, pimaricin, neomycin sulfate, chloramphenicol, bacitracin, sulfacetamide, gentamycin, polymixin B sulfate, and the like. The composition is applied to the patient's eye in sufficient amounts to deliver a pharmacologically effective amount.

Suitable vasoconstricting (i.e. decongesting) agents include, for example, 4,5-dihydro-2-(1,2,3,4-tetrahydro-1-naphthylenyl)-1H-imidazole, also known as tetrahydrozoline.

The present invention also includes methods for improving the comfort of wearers of hydrogel contact lenses comprising treating the eyes of such wearers with the inventive solutions described above. The administration of the solution to the eyes may be by any suitable method, for example in the form of drops from a standard applicator. Treatments are preferably repeated several times daily, such as 2 to 5 times. The number of drops depends on the concentration of the solution. Typically, 1-5 drops are suitable.

Having generally described the invention, a more complete understanding can be obtained by reference to certain specific examples, which are provided for purposes of illustration only and are not intended to be limiting unless otherwise specified.

Example 1

The following solutions are suitable for use in this invention.

```
(a) 0.2 %   Boric acid                            } Borate Buffer
    0.33 %  Sodium Tetraborate Decahydrate        } pH 7.6
    0.2 %   Ethylene Diamine Tetra Acetate (EDTA)
    0.1 %   Sorbic Acid
    0.7 %   Hydroxyethylcellulose
    0.2 %   Poloxamer 407
    1.14 %  Urea


(b) 0.20 %  Boric Acid                            } Borate Buffer
    0.33 %  Sodium Tetraborate Decahydrate        } pH 7.6
    0.25 %  EDTA
    0.15 %  Sorbic Acid
    1.0 %   Hydroxyethylcellulose
    0.8 %   Tyloxapol
    0.25 %  Urea
    1.41 %  Sodium Chloride
```

```
(c)  0.37 %  Boric Acid                          } Borate Buffer
     0.06 %  Sodium Tetraborate Decahydrate       } pH 7.4
     0.2 %   EDTA
     0.1 %   Sorbic Acid
     0.7 %   Hydroxyethylcellulose
     0.76 %  Glycerol


(d)  0.2 %   Boric Acid                          } Borate Buffer
     0.33 %  Sodium Tetraborate Decahydrate       } pH 7.6
     0.2 %   EDTA
     0.1 %   Sorbic Acid
     0.7 %   Hydroxyethylcellulose
     0.2 %   Poloxamer 407
     0.001 % Urea
     1.56 %  Sodium Chloride


(e)  0.16 %  Boric Acid                          } Borate Buffer
     0.38 %  Sodium Tetraborate Decahydrate       } pH 7.3
     0.2 %   Disodium EDTA
     0.15 %  Sorbic Acid
     0.7 %   Hydroxyethylcellulose
     0.2 %   Poloxamer 407
     0.03 %  Urea
     0.52 %  Sodium Chloride


(f)  0.16 %  Boric Acid                          } Borate Buffer
     0.38 %  Sodium Tetraborate Decahydrate       } pH 7.3
     0.2 %   Disodium EDTA
     0.15 %  Sorbic Acid
     0.7 %   Hydroxyethylcellulose
     0.2 %   Poloxamer 407
     0.02 %  Urea
     0.44 %  Sodium Chloride
```

Example 2:

The flattening of the lens is illustrated in vitro by measuring the base curve diameter (D) with a Nikon Profile Projector Model V-12 and the sagital height (S) with a Jones and Lamson Profile Projector. A lower ratio of S to D implies a flatter lens.

| Results: | | | | | | |
|---|---|---|---|---|---|---|
| Lens | Run | Before Treatment | | | After treatment with aqueous solution of 0.56 % urea | | |
| | | D (mm) | S (mm) | S/D | D (mm) | S (mm) | S/D |
| AO-SO[1] | 1 | 14.296 | 4.196 | 0.294 | 15.102 | 3.606 | 0.239 |
| | 2 | 14.059 | 4.127 | 0.294 | 15.051 | 3.633 | 0.241 |
| Hydrocurve II[2] | 1 | 14.325 | 4.083 | 0.285 | 16.404 | 4.013 | 0.245 |
| | 2 | 14.210 | 4.077 | 0.287 | 17.225 | 4.343 | 0.252 |
| CibaSoft[3] | 1 | 13.755 | 3.578 | 0.260 | 14.182 | 3.660 | 0.258 |
| | 2 | 14.301 | 3.573 | 0.259 | 14.301 | 3.613 | 0.253 |

(1) The lens is the AO-SO soft contact lens of the American Optical Company, and contains a copolymer of hydroxyethyl methacrylate and polyvinyl pyrrolidone and 55 % water.
(2) The lens is the Hydrocurve II soft contact lens of Barns-Hind and contains a copolymer of hydroxyethyl methacrylate and polyvinyl pyrrolidone and 45 % water.
(3) The lens is the CibaSoft contact lens of Ciba Vision Care and contains hydroxyethyl methacrylate and 38 % water.

## Example 3

Solutions suitable for use with various lenses.

### Spherical daily wear hydrogel lenses

| Trade Name | Manufacturer | Material | % H$_2$O | Thickness (mm) | Diameters (mm) | Base Curves (mm) | Powers (diopters) | Solution [1] |
|---|---|---|---|---|---|---|---|---|
| AOSOFT | American Optical | HEMA/NVP/ MMA | 42.5 | .12 | 13.0 | 7.8 to 9.0 (.3) | -10 to +6.50 | D |
|  |  |  |  | .04 | 13.8 | 8.3, 8.6, 8.9 | -7.75 to PLANO | D |
| AQUAFLEX | Coopervision | HEMA/NVP/ MMA | 42.5 | .10 | 13.0 | 7.8 to 9.0 (.3) | -9.75 to PLANO | D |
|  |  |  |  | .05 | 13.8 | 8.2 to 9.1 (.3) | -20 to +10 | D |
|  |  |  |  | .42 | 13.8 | 8.2 to 9.4 (.3) | +10 to +20 | D |
| CIBASOFT | Ciba | HEMA | 37.5 | .09 | 13.8 | 8.3 to 9.2 (.3) | -6 to PLANO | D |
|  |  |  |  | .09 | 14.5 | 8.6, 8.9, 9.2 | -10 to PLANO | D |
|  |  |  |  | .03 | 13.8 | 8.3, 8.6, 8.9 | -6 to -1 | D or E |
| C.S.I | Syntex | GMA/MMA | 38.5 | .05 | 13.8 | 8.0, 8.3, 8.6 | -20 to +8 | D |
|  |  |  |  | .05 | 14.8 | 8.6, 8.9, 9.35 | -20 to PLANO | D |
| CUSTOM EYES | C.T.L. | HEMA (Tinted) | 38 | .06 | 14.0 | 8.4, 8.7 | -8 to +5 | D |
|  |  |  |  | .07 | 13.5,14.5 | Spin Cast | -7 to +5 | D |
|  |  |  |  | .11 | 14.5 | Spin Cast | -2 to PLANO | D |
| HYDROCURVE | Barnes-Hind | HEMA/ Acrylamide | 45 | .05 | 13.5 | 8.3, 8.6 | -12 to +7 | D |
|  |  |  |  | .05 | 14.5 | 8.9 | -12 to +7 | D |
|  |  |  |  | .05 | 15.5,16.0 | 9.2 to 10.1 (.3) | -20 to +20 | D |
| HYDRON | American Hydron | HEMA | 38 | .12 | 13.0 | 8.1 to 9.1 (.2) | -20 to +8 | D |
|  |  |  |  | .06 | 14.0 | 8.4, 8.7, 9.0 | -10 to PLANO | D |
|  |  |  |  | .04 | 13.8 | 8.6 | -6 to PLANO |  |
| HYDROSIGHT | National Contact Lenses | HEMA/BMA | 43 | .14 | 13.5 | 7.8 to 9.2 (.2) | -10 to +5 | D |
|  |  |  |  | .06 | 13.8 | 8.3, 8.6, 8.9 | -12 to PLANO | D |
| NATURAL TINT | Bausch & Lomb | HEMA (Tinted) | 38 |  | 13.5 |  | -6 to PLANO | D |
|  |  |  |  |  | 14.5 |  | -6 to PLANO | D |

EP 0 198 490 B1

| Trade Name | Manufacturer | Material | % H₂O | Thickness (mm) | Diameters (mm) | Base Curves (mm) | Powers (diopters) | Solution [1] |
|---|---|---|---|---|---|---|---|---|
| P.D.C. | P.D.C., Inc. | HEMA | 38 | .06 | 14.0 | 8.3 to 8.9 (.2) | | D |
| SAUFLON 70 | Vision Tech | MMA/NVP | 70 | | 14.3 | 8.4, 8.7, 9.0 | -8 to PLANO | E |
| SOFLENS | Bausch & Lomb | HEMA | 38 | | 13.5 | $B_3,U_3,O_3,HO_3,L_3,H_3$ | -20 to +20 | D or E[2] |
| | | | | | 14.5 | $B_4,U_4,O_4,HO_4,L_4,H_4$ | -20 to +20 | D or E[2] |
| SOFT COLORS | Ciba | HEMA (Tinted) | 37.5 | .09 | 13.8 | 8.3 to 9.2 (.3) | -6 to PLANO | D |
| | | | | .09 | 14.5 | 8.6, 8.9, 9.2 | -10 to PLANO | D |
| SOFTCON | American Optical | HEMA/PVP | 55 | .10 | 14.0,14.5 | 7.8 to 8.7 (.3) | -8 to +18 | D or E[2] |

Toric Daily Wear Hydrogel Lenses

| Trade Name | Manufacturer | Material | % H₂O | Diameters (mm) | Base Curves (mm) | Spheres | Cylinder (diopters) | Axis | Ballast | Solution[1] |
|---|---|---|---|---|---|---|---|---|---|---|
| B & L TORIC | Bausch & Lomb | HEMA/NVP/ | 45 | 14.0 | 8.3, 8.6 | -6 to +4 | -.75, -1.25 -1.75 | 90°(+20°) 180°(±20°) | prism | D |
| HYDROCURVE II TORIC | Barnes-Hind | | 45 55* | 13.5 14.5 | 8.3, 8.6 8.8 | -6 to +3 -6 to +3 | -1.25, -2.00 -1.25, -2.00 | 180° +25° 90° ±25° | prism | E |
| TORI-SOFT | Ciba | HEMA | 37.5 | 14.5 | 8.6,8.9,9.2 | -6 to +4 | -1, -1.75 | 180° ±20° 90° ±20° | thin zones | D |

* Also approved for extended wear

EP 0 198 490 B1

## Multifocal Soft Lenses

| Trade Name | Manufacturer | Material | % $H_2O$ | Diameters (mm) | Base Curves (mm) | Powers (diopters) | Adds | Solution [1] |
|---|---|---|---|---|---|---|---|---|
| AO MULTIVUE | American Optical | HEMA | 38 | 13.8 | 8.3, 8.6, 8.9 | | | D |
| BI-SOFT | Ciba | HEMA | 38 | 13.8 | 8.3, 8.6, 8.9 | -8 to +8 | +1.50 +3.00 | D |

## Aphakic Extended Wear

| Trade Name | Manufacturer | Material | % $H_2O$ | Diameters (mm) | Base Curves (mm) | Powers (diopters) | Solution [1] |
|---|---|---|---|---|---|---|---|
| C.W. 79 | Bausch & Lomb | MMA/NVP | 79 | 14.4 | 8.1, 8.4, 8.7 | +10 to +20 | E |
| HYDROCURVE II | Barnes-Hind | HEMA/ Acrylamide | 45 | 13.5 | 8.3, 8.6, 8.9 | +7 to +20 | E |
| HYDROCURVE II55 | Barnes-Hind | HEMA/ Acrylamide | 55 | 14.0 | 8.5 | | E |
| | | | | 14.5 | 8.8 | +7 to +20 | E |
| | | | | 15.5, 16.0 | 9.2 to 10.1(.3) | | E |
| PERMALENS | Cooper Vision | | 71 | 14.0, 14.5 | 8.0 to 8.9 (.3) | +8 to +20 | E |
| SAUFLON P.W. | Vision Tech | MMA/NVP | 79 | 14.4 | 8.1, 8.4, 8.7 | PLANO to +20 | E |
| | | | | 13.7 | 7.5, 7.8 | +20 to +35 | E |
| SOFTCON | American Optical | HEMA/PVP | 55 | 14.0, 14.5 | 7.8 to 8.7 (.3) | +8 to +18 | D or E[2] |

EP 0 198 490 B1

## Cosmetic Extended Wear

| Trade Name | Manufacturer | Material | % H$_2$O | Diameters (mm) | Base Curves (mm) | Powers (diopters) | Solution [1] |
|---|---|---|---|---|---|---|---|
| B & L 70 | Bausch & Lomb | MMA/NVP | 70 | 14.3 | 8.4, 8.7, 9.0 | -6 to PLANO | E |
| O$_3$, O$_4$ | Bausch & Lomb | HEMA | 38 | 13.5,14.0 | Spin Cast | -6 to PLANO | D or E[2] |
| CSI-T | Syntex | GMA | 38 | 13.8 | 8.3, 8.6 | -7 to PLANO | D |
|  |  |  |  | 14.8 | 8.6, 8.9, 9.3 | -7 to PLANO | D |
| HYDROCURVE II$_{55}$ | Barnes-Hind | HEMA/ Acrylamide | 55 | 14.0 | 8.5 | -12 to PLANO | D or E[2] |
|  |  |  |  | 14.5 | 8.8 | -12 to PLANO | D or E[2] |
| HYDROCURVE II TORIC (SEE TORIC SECTION) |  |  |  |  |  |  |  |
| GENESIS IV | Channel | MMA/NVP | 70 | 14.3 | 8.4, 8.7, 9.0 | -8 to PLANO | E |
| HYDROSIGHT 70 | National | MMA/NVP | 70 | 14.3 | 8.4, 8.7, 9.0 | -8 to +6 | E |
| PDC 70 | P.D.C. | MMA/NVP | 70 | 14.4 | 8.7, 9.0 | -8 to PLANO | E |
| PERMALENS | Cooper Vision |  | 71 | 13.5,14.2 | 7.7 to 8.6 (.3) | -20 to PLANO | E |
|  |  |  |  | 14.0 | 7.7 to 8.6 (.3) | PLANO to +8 | E |
| PERMAFLEX | Cooper Vision |  | 74 | 14.4 | 8.7 | -12 to +8 | E |
| SAUFLON 70 | Vision Tech | MMA/NVP | 70 | 14.3 | 8.4, 8.7, 9.0 | -12 to +8 | E |
| SOFTCON | American Optical | HEMA/PVP | 55 | 14.0,14.5 | 7.8 to 8.7 (.3) | -8 to +8 | D or E |
| CSI | Syntex | GMA | 38 | 14.8 | 8.6, 8.9, 9.35 | -7 to PLANO | D |
| SAUFLON PW | Vision Tech | MMA/NVP | 79 | 14.4 | 8.1, 8.4, 8.7 | PLANO to +20 | E |
| SOFLENS | Bausch & Lomb | HEMA | 38 | 12.5 | U |  |  |
|  |  |  |  | 13.5 | U$_3$ | -9 to PLANO | E |
|  |  |  |  | 14.5 | O$_4$, B$_4$ |  |  |
| SOFTCON | American Optical |  | 55 | 14, 14.5 | 7.8 to 8.7 (.3) | -8 to +18 | D or E |

EP 0 198 490 B1

EP 0 198 490 B1

1. D represents a solution suitable for a daily wear lens as described in the specification or in example 1(b).

E represents a solution suitable for an extended wear lens as described in the specification or in example 1(d).

2. Preferred

In the above table the abbreviations for the materials are

BMA = Butyl methacrylate

GMA = Glyceryl methacrylate

HEMA = Hydroxyethyl methacrylate

MMA = Methyl methacrylate

NVP = N-Vinylpyrrolidone

PVP = Polyvinylpyrrolidone

## Claims
## Claims for the following Contracting States : BE, CH, LI, DE, FR, GB, IT, LU, NL, SE

1. An ophthalmic comfort drop solution for intra ocular use with a daily wear hydrogel contact lens for improving the exchange of
   I) ocular fluid trapped between said lens and an eye to which said lens has been applied for
   II) ocularly compatible fluid not so trapped, said solution comprising:
   a) 0.1 to 0.5 % by weight of a lens flattening agent selected from urea, glycerin, sorbitol, aminoethanol and mixtures thereof; provided that the amount of glycerin is below 0.4 % by weight;
   b) an ocularly acceptable ionic salt in an amount sufficient to give the entire comfort drop solution a tonicity in the range of 270 - 330 milliosmoles per kilogram of said comfort drop solution;
   c) an ocularly acceptable viscosity enhancing agent in an amount sufficient to give said comfort drop solution a Brookfield relative viscosity in the range of 3 to 50 mPa$\cdot$s (cps);
   d) an ocularly acceptable buffer in an amount of 0 % or an effective buffering amount to result in said comfort drop solution having a pH of 6.5 to 8;
   e) 0 % to 0.5 % of an ocularly acceptable preservative;
   f) 0 % to 2 % of an ocularly acceptable lubricant;
   g) 0 % or an ocularly acceptable pharmaceutically effective amount of an ocularly administrable pharmaceutically active agent; and
   h) 0 % to 1 % of a non-ionic ocularly acceptable surfactant.

2. The comfort drop solution of claim 1 wherein said flattening agent is urea.

3. The comfort drop solution of claim 1 wherein said flattening agent is present in an amount of 0.1 % to 0.4% by weight.

4. The comfort drop solution of claim 1 wherein said flattening agent is present in an amount of 0.15 % to 0.3% by weight.

5. The comfort drop solution of claim 1 wherein said Brookfield relative viscosity is in the range of from 3 to 20 mPa$\cdot$s (cps).

13

**6.** The comfort drop solution of claim 5 wherein said Brookfield relative viscosity is in the range of from 6 to 11 mPa•s (cps).

**7.** The comfort drop solution of claim 1 wherein said viscosity enhancing agent is hydroxyethyl cellulose.

**8.** The comfort drop solution of claim 1 wherein said ionic salt is present in at least an amount which is equivalent in osmolarity to a solution of 0.3 % by weight sodium chloride.

**9.** The comfort drop solution of claim 1 wherein said ionic salt is present in at least an amount which is equivalent in osmolarity to a solution of 0.5 % by weight sodium chloride.

**10.** The comfort drop solution of claim 1 wherein said ionic salt is present in at least an amount which is equivalent in osmolarity to a solution of 0.75 % by weight sodium chloride.

**11.** The comfort drop solution of claim 1 wherein said ionic salt is sodium chloride.

**12.** The comfort drop solution of claim 1 wherein said preservative is ocularly non-irritating.

**13.** The comfort drop solution of claim 1 wherein said preservative is present in an amount of from 0.001 to 0.5 % by weight of said comfort drop.

**14.** The comfort drop solution of claim 1 wherein said preservative is present in an amount of 0.1 to 0.25 % by weight of said comfort drop.

**15.** The comfort drop solution of claim 1 wherein said preservative is sorbic acid.

**16.** The comfort drop solution of claim 1 wherein said pH of said comfort drop is from 7.0 to 7.6.

**17.** The comfort drop solution of claim 1 wherein said pH of said comfort drop is 7.3.

**18.** The comfort drop solution of claim 1 wherein said buffer is a borate buffer.

**19.** The comfort drop solution of claim 1 wherein said ocularly administrable pharmaceutically active agent is an ocular decongestant and is present in an ocularly vasoconstricting effective amount.

**20.** The comfort drop solution of claim 1 comprising 0.2 % by weight urea; 0.44 % by weight NaCl; 0.16 % by weight Boric Acid; 0.38 % by weight Na tetraborate decahydrate; 0.2 % by weight disodium EDTA; 0.15 % by weight sorbic acid; 0.7 % by weight hydroxyethylcellulose; 0.2 % by weight of an ethylene oxide-propylene oxide copolymer such as Poloxamer® 407.

**21.** An ophthalmic comfort drop solution for intra ocular use with an extended wear hydrogel contact lens for improving the exchange of
   I) ocular fluid trapped between said lens and an eye to which said lens has been applied for
   II) ocularly compatible fluid not so trapped, said solution comprising:
      a) 0.0005 to 0.1 % by weight of a lens flattening agent selected from urea, glycerin, sorbitol, aminoethanol and mixtures thereof;
      b) an ocularly acceptable ionic salt in an amount sufficient to give the entire comfort drop solution a tonicity in the range of 270 - 330 milliosmoles per kilogram of said comfort drop solution;
      c) an ocularly acceptable viscosity enhancing agent in an amount sufficient to give said comfort drop solution a Brookfield relative viscosity in the range of 3 to 50 mPa•s (cps);
      d) an ocularly acceptable buffer in an amount of 0 % or an effective buffering amount to result in said comfort drop solution having a pH of 6.5 to 8;
      e) 0 % to 0.5 % of an ocularly acceptable preservative;
      f) 0 % to 2 % of an ocularly acceptable lubricant;
      g) 0 % or an ocularly acceptable pharmaceutically effective amount of an ocularly administrable pharmaceutically active agent; and
      h) 0 % to 1 % of a non-ionic ocularly acceptable surfactant.

**22.** The comfort drop solution of claim 21 wherein said flattening agent is urea.

**23.** The comfort drop solution of claim 21 wherein said flattening agent is present in an amount of 0.001 % to 0.08 % by weight.

**24.** The comfort drop solution of claim 21 wherein said flattening agent is present in an amount of 0.01 % to 0.05 % by weight.

**25.** The comfort drop solution of claim 21 wherein said Brookfield relative viscosity is in the range of from 3 to 20 mPa•s (cps).

**26.** The comfort drop solution of claim 25 wherein said Brookfield relative viscosity is in the range of from 6 to 11 mPa•s (cps).

**27.** The comfort drop solution of claim 21 wherein said viscosity enhancing agent is hydroxyethyl cellulose.

**28.** The comfort drop solution of claim 21 wherein said ionic salt is present in at least an amount which is equivalent in osmolarity to a solution of 0.3 % by weight sodium chloride.

**29.** The comfort drop solution of claim 21 wherein said ionic salt is present in at least an amount which is equivalent in osmolarity to a solution of 0.5 % by weight sodium chloride.

**30.** The comfort drop solution of claim 21 wherein said ionic salt is present in at least an amount which is equivalent in osmolarity to a solution of 0.75 % by weight sodium chloride.

**31.** The comfort drop solution of claim 21 wherein said ionic salt is sodium chloride.

**32.** The comfort drop solution of claim 21 wherein said preservative is ocularly non-irritating.

**33.** The comfort drop solution of claim 21 wherein said preservative is present in an amount of from 0.001 to 0.5 % by weight of said comfort drop.

**34.** The comfort drop solution of claim 21 wherein said preservative is present in an amount of 0.1 to 0.25 % by weight of said comfort drop.

**35.** The comfort drop solution of claim 21 wherein said preservative is sorbic acid.

**36.** The comfort drop solution of claim 21 wherein said pH of said comfort drop is from 7.0 to 7.6.

**37.** The comfort drop solution of claim 21 wherein said pH of said comfort drop is 7.3.

**38.** The comfort drop solution of claim 21 wherein said buffer is a borate buffer.

**39.** The comfort drop solution of claim 21 wherein said ocularly administrable pharmaceutically active agent is an ocular decongestant and is present in an ocularly vasoconstricting effective amount.

**40.** The comfort drop solution of claim 21 comprising 0.03 % by weight urea; 0.52 % by weight NaCl; 0.16 % by weight Boric Acid; 0.38 % by weight Na tetraborate decahydrate; 0.2 % by weight disodium EDTA; 0.15 % by weight sorbic acid; 0.7 % by weight hydroxyethylcellulose; 0.2 % by weight of an ethylene oxide-propyleneoxide copolymer such as Poloxamer® 407.

**41.** Use of a comfort drop solution comprising:
a) 0.1 to 0.5 % by weight of a lens flattening agent selected from urea, glycerin, sorbitol, aminoethanol and mixtures thereof;
b) an ocularly acceptable ionic salt in an amount sufficient to give the entire comfort drop solution a tonicity in the range of 270 - 330 milliosmoles per kilogram of said comfort drop solution;
c) an ocularly acceptable viscosity enhancing agent in an amount sufficient to give said comfort drop solution a Brookfield relative viscosity in the range of 3 to 50 mPa•s (cps);

d) an ocularly acceptable buffer in an amount of 0 % or an effective buffering amount to result in said comfort drop solution having a pH of 6.5 to 8;

e) 0 % to 0.5 % of an ocularly acceptable preservative;

f) 0 % to 2 % of an ocularly acceptable lubricant;

g) 0 % of an ocularly administrable pharmaceutically active agent; and

h) 0 % to 1 % of a non-ionic ocularly acceptable surfactant

for improving the exchange of

I) fluid located between a daily wear hydrogel contact lens and an eye to which said lens has been applied, with

II) an ocularly compatible fluid not so located, while said lens is being worn on said eye

comprising instilling to said eye, having said lens thereon, a fluid exchange improving effective amount of said comfort drop solution, whereby said lens is temporarily flattened allowing for greater fluid exchange than is possible in the absence of said comfort drop solution.

**42.** The use of claim 41 wherein said comfort drop solution is instilled in an amount of 1 to 5 drops up to 5 times daily.

**43.** Use of a comfort drop solution of claim 20 for improving the exchange of

I) fluid located between a daily wear hydrogel contact lens and an eye to which said lens has been applied, with

II) an ocularly compatible fluid not so located, while said lens is being worn on said eye

comprising instilling to said eye, having said lens thereon, a fluid exchange improving effective amount of said comfort drop solution of claim 20, whereby said lens is temporarily flattened allowing for greater fluid exchange than is possible in the absence of said comfort drop solution of claim 20.

**44.** The use of claim 43 wherein said comfort drop solution is instilled in an amount of 1 to 5 drops up to 5 times daily.

**45.** Use of a comfort drop solution of claim 21 wherein component g) is absent for improving the exchange of

I) fluid located between an extended wear hydrogel contact lens and an eye to which said lens has been applied, with

II) an ocularly compatible fluid not so located, while said lens is being worn on said eye

comprising instilling to said eye, having said lens thereon, a fluid exchange improving effective amount of said comfort drop solution of claim 21, whereby said lens is temporarily flattened allowing for greater fluid exchange than is possible in the absence of said comfort drop solution of claim 21.

**46.** The use of claim 45 wherein said comfort drop solution is instilled in an amount of 1 to 5 drops up to 5 times daily.

**47.** Use of a comfort drop solution of claim 40 for improving the exchange of

I) fluid located between an extended wear hydrogel contact lens and an eye to which said lens has been applied, with

II) an ocularly compatible fluid not so located, while said lens is being worn on said eye

comprising instilling to said eye, having said lens thereon, a fluid exchange improving effective amount of said comfort drop solution of claim 40, whereby said lens is temporarily flattened allowing for greater fluid exchange than is possible in the absence of said comfort drop solution of claim 40.

**48.** The use of claim 47 wherein said comfort drop solution is instilled in an amount of 1 to 5 drops up to 5 times daily.

**Claims for the following Contracting State : AT**

**1.** A process for the manufacture of an ophthalmic comfort drop solution for intra ocular use with a daily wear hydrogel contact lens for improving the exchange of

I) ocular fluid trapped between said lens and an eye to which said lens has been applied for

II) ocularly compatible fluid not so trapped, said solution comprising:

0.1 to 0.5 % by weight of a lens flattening agent selected from urea, glycerin, sorbitol, aminoethanol and mixtures thereof; provided that the amount of glycerin is below 0.4 % by weight;

b) an ocularly acceptable ionic salt in an amount sufficient to give the entire comfort drop solution a tonicity in the range of 270 - 330 milliosmoles per kilogram of said comfort drop solution;

c) an ocularly acceptable viscosity enhancing agent in an amount sufficient to give said comfort drop solution a Brookfield relative viscosity in the range of 3 to 50 mPa•s (cps);

d) an ocularly acceptable buffer in an amount of 0 % or an effective buffering amount to result in said comfort drop solution having a pH of 6.5 to 8;

e) 0 % to 0.5 % of an ocularly acceptable preservative;

f) 0 % to 2 % of an ocularly acceptable lubricant;

g) 0 % or an ocularly acceptable pharmaceutically effective amount of an ocularly administrable pharmaceutically active agent; and

h) 0 % to 1 % of a non-ionic ocularly acceptable surfactant; by conventionally mixing the components.

2. The process of claim 1 wherein said flattening agent is urea.

3. The process of claim 1 wherein said flattening agent is present in an amount of 0.1 % to 0.4 % by weight.

4. The process of claim 1 wherein said flattening agent is present in an amount of 0.15 % to 0.3 % by weight.

5. The process of claim 1 wherein said Brookfield relative viscosity is in the range of from 3 to 20 mPa•s (cps).

6. The process of claim 5 wherein said Brookfield relative viscosity is in the range of from 6 to 11 mPa•s (cps).

7. The process of claim 1 wherein said viscosity enhancing agent is hydroxyethyl cellulose.

8. The process of claim 1 wherein said ionic salt is present in at least an amount which is equivalent in osmolarity to a solution of 0.3 % by weight sodium chloride.

9. The process of claim 1 wherein said ionic salt is present in at least an amount which is equivalent in osmolarity to a solution of 0.5 % by weight sodium chloride.

10. The process of claim 1 wherein said ionic salt is present in at least an amount which is equivalent in osmolarity to a solution of 0.75 % by weight sodium chloride.

11. The process of claim 1 wherein said ionic salt is sodium chloride.

12. The process of claim 1 wherein said preservative is ocularly nonirritating.

13. The process of claim 1 wherein said preservative is present in an amount of from 0.001 to 0.5 % by weight of said comfort drop.

14. The process of claim 1 wherein said preservative is present in an amount of 0.1 to 0.25 % by weight of said comfort drop.

15. The process of claim 1 wherein said preservative is sorbic acid.

16. The process of claim 1 wherein said pH of said comfort drop is from 7.0 to 7.6.

17. The process of claim 1 wherein said pH of said comfort drop is 7.3.

18. The process of claim 1 wherein said buffer is a borate buffer.

17

**19.** The process of claim 1 wherein said ocularly administrable pharmaceutically active agent is an ocular decongestant and is present in an ocularly vasoconstricting effective amount.

**20.** The process of claim 1 wherein said comfort drop solution is comprising 0.2 % by weight urea; 0.44 % by weight NaCl; 0.16 % by weight Boric Acid; 0.38 % by weight Na tetraborate decahydrate; 0.2 % by weight disodium EDTA; 0.15 % by weight sorbic acid; 0.7 % by weight hydroxyethylcellulose; 0.2 % by weight of an ethylene oxidepropylene oxide copolymer such as Poloxamer® 407.

**21.** A process for the manufacture of an ophthalmic comfort drop solution for intra ocular use with an extended wear hydrogel contact lens for improving the exchange of

I) ocular fluid trapped between said lens and an eye to which said lens has been applied for

II) ocularly compatible fluid not so trapped, said solution comprising:

a) 0.0005 to 0.1 % by weight of a lens flattening agent selected from urea, glycerin, sorbitol, aminoethanol and mixtures thereof;

b) an ocularly acceptable ionic salt in an amount sufficient to give the entire comfort drop solution a tonicity in the range of 270 - 330 milliosmoles per kilogram of said comfort drop solution;

c) an ocularly acceptable viscosity enhancing agent in an amount sufficient to give said comfort drop solution a Brookfield relative viscosity in the range of 3 to 50 mPa•s (cps);

d) an ocularly acceptable buffer in an amount of 0 % or an effective buffering amount to result in said comfort drop solution having a pH of 6.5 to 8;

e) 0 % to 0.5 % of an ocularly acceptable preservative;

f) 0 % to 2 % of an ocularly acceptable lubricant;

g) 0 % or an ocularly acceptable pharmaceutically effective amount of an ocularly administrable pharmaceutically active agent; and

h) 0 % to 1 % of a non-ionic ocularly acceptable surfactant;

by conventionally mixing the components.

**22.** The process of claim 21 wherein said flattening agent is urea.

**23.** The process of claim 21 wherein said flattening agent is present in an amount of 0.001 % to 0.08 % by weight.

**24.** The process of claim 21 wherein said flattening agent is present in an amount of 0.01 % to 0.05 % by weight.

**25.** The process of claim 21 wherein said Brookfield relative viscosity is in the range of from 3 to 20 mPa•s (cps).

**26.** The process of claim 25 wherein said Brookfield relative viscosity is in the range of from 6 to 11 mPa•s (cps).

**27.** The process of claim 21 wherein said viscosity enhancing agent is hydroxyethyl cellulose.

**28.** The process of claim 21 wherein said ionic salt is present in at least an amount which is equivalent in osmolarity to a solution of 0.3 % by weight sodium chloride.

**29.** The process of claim 21 wherein said ionic salt is present in at least an amount which is equivalent in osmolarity to a solution of 0.5 % by weight sodium chloride.

**30.** The process of claim 21 wherein said ionic salt is present in at least an amount which is equivalent in osmolarity to a solution of 0.75 % by weight sodium chloride.

**31.** The process of claim 21 wherein said ionic salt is sodium chloride.

**32.** The process of claim 21 wherein said preservative is ocularly non-irritating.

**33.** The process of claim 21 wherein said preservative is present in an amount of from 0.001 to 0.5 % by weight of said comfort drop.

18

**34.** The process of claim 21 wherein said preservative is present in an amount of 0.1 to 0.25 % by weight of said comfort drop.

**35.** The process of claim 21 wherein said preservative is sorbic acid.

**36.** The process of claim 21 wherein said pH of said comfort drop is from 7.0 to 7.6.

**37.** The process of claim 21 wherein said pH of said comfort drop is 7.3.

**38.** The process of claim 21 wherein said buffer is a borate buffer.

**39.** The process of claim 21 wherein said ocularly administrable pharmaceutically active agent is an ocular decongestant and is present in an ocularly vasoconstricting effective amount.

**40.** The process of claim 21 wherein said comfort drop solution is comprising 0.03 % by weight urea; 0.52 % by weight NaCl; 0.16 % by weight Boric Acid; 0.38 % by weight Na tetraborate decahydrate; 0.2 % by weight disodium EDTA; 0.15 % by weight sorbic acid; 0.7 % by weight hydroxyethylcellulose; 0.2 % by weight of an ethylene oxidepropyleneoxide copolymer such as Poloxamer® 407; and water.

**41.** Use of a comfort drop solution comprising:
a) 0.1 to 0.5 % by weight of a lens flattening agent selected from urea, glycerin, sorbitol, aminoethanol and mixtures thereof;
b) an ocularly acceptable ionic salt in an amount sufficient to give the entire comfort drop solution a tonicity in the range of 270 - 330 milliosmoles per kilogram of said comfort drop solution;
c) an ocularly acceptable viscosity enhancing agent in an amount sufficient to give said comfort drop solution a Brookfield relative viscosity in the range of 3 to 50 mPa•s (cps);
d) an ocularly acceptable buffer in an amount of 0 % or an effective buffering amount to result in said comfort drop solution having a pH of 6.5 to 8;
e) 0 % to 0.5 % of an ocularly acceptable preservative;
f) 0 % to 2 % of an ocularly acceptable lubricant;
g) 0 % of an ocularly administrable pharmaceutically active agent; and
h) 0 % to 1 % of a non-ionic ocularly acceptable surfactant
for improving the exchange of
I) fluid located between a daily wear hydrogel contact lens and an eye to which said lens has been applied, with
II) an ocularly compatible fluid not so located, while said lens is being worn on said eye

comprising instilling to said eye, having said lens thereon, a fluid exchange improving effective amount of said comfort drop solution, whereby said lens is temporarily flattened allowing for greater fluid exchange than is possible in the absence of said comfort drop solution.

**42.** The use of claim 41 wherein said comfort drop solution is instilled in an amount of 1 to 5 drops up to 5 times daily.

**43.** Use of a comfort drop solution as defined in claim 20 for improving the exchange of
I) fluid located between a daily wear hydrogel contact lens and an eye to which said lens has been applied, with
II) an ocularly compatible fluid not so located, while said lens is being worn on said eye
comprising instilling to said eye, having said lens thereon, a fluid exchange improving effective amount of said comfort drop solution of claim 20, whereby said lens is temporarily flattened allowing for greater fluid exchange than is possible in the absence of said comfort drop solution of claim 20.

**44.** The use of claim 43 wherein said comfort drop solution is instilled in an amount of 1 to 5 drops up to 5 times daily.

**45.** Use of a comfort drop solution as defined in claim 21 wherein component g) is absent for improving the exchange of
I) fluid located between an extended wear hydrogel contact lens and an eye to which said lens has been applied, with

II) an ocularly compatible fluid not so located, while said lens is being worn on said eye comprising instilling to said eye, having said lens thereon, a fluid exchange improving effective amount of said comfort drop solution of claim 21, whereby said lens is temporarily flattened allowing for greater fluid exchange than is possible in the absence of said comfort drop solution of claim 21.

46. The use of claim 45 wherein said comfort drop solution is instilled in an amount of 1 to 5 drops up to 5 times daily.

47. Use of a comfort drop solution as defined in claim 40 for improving the exchange of

I) fluid located between an extended wear hydrogel contact lens and an eye to which said lens has been applied, with

II) an ocularly compatible fluid not so located, while said lens is being worn on said eye comprising instilling to said eye, having said lens thereon, a fluid exchange improving effective amount of said comfort drop solution of claim 40, whereby said lens is temporarily flattened allowing for greater fluid exchange than is possible in the absence of said comfort drop solution of claim 40.

48. The use of claim 47 wherein said comfort drop solution is instilled in an amount of 1 to 5 drops up to 5 times daily.

**Revendications**
**Revendications pour les Etats contractants suivants : BE, CH, DE, FR, GB, IT, LI, LU, NL, SE**

1. Solution de gouttes de confort ophtalmique pour utilisation intraoculaire avec une lentille de contact d'hydrogel à utilisation quotidienne pour améliorer l'échange

I) Du fluide oculaire piégé entre ladite lentille et un oeil auquel ladite lentille a été appliquée pour

II) Un fluide oculairement compatible non ainsi piégé, ladite solution comprenant :

a) de 0,1 à 0,5% en poids d'un agent aplatissant de lentille choisi parmi l'urée, la glycérine, le sorbitol, l'aminoéthanol et leurs mélanges ; à condition que la quantité de glycérine soit inférieure à 0,4% en poids ;

b) un sel ionique oculairement acceptable en une quantité suffisante pour donner à la totalité de la solution de gouttes de confort une tonicité située dans un intervalle de 270-330 milliosmoles/kg de ladite solution de gouttes de confort ;

c) un agent améliorant la viscosité oculairement acceptable en une quantité suffisante pour donner à ladite solution de gouttes de confort une viscosité relative de Brookfield située dans un intervalle allant de 3 à 50 mPa.s (cps) ;

d) un tampon oculairement acceptable en une quantité de 0% ou une quantité efficace comme tampon pour que ladite solution de gouttes de confort ait un pH de 6,5 à 8 ;

e) de 0% à 0,5% d'un agent de conservation oculairement acceptable ;

f) de 0% à 2% d'un lubrifiant oculairement acceptable ;

g) 0% ou une quantité pharmaceutiquement efficace et oculairement acceptable d'un agent pharmaceutiquement actif administrable par voie oculaire ; et

h) de 0% à 1% d'un agent tensio-actif oculairement acceptable non ionique.

2. Solution de gouttes de confort de la revendication 1, dans laquelle l'agent aplatissant est l'urée.

3. Solution de gouttes de confort de la revendication 1, dans laquelle ledit agent aplatissant est présent en une quantité de 0,1% à 0,4% en poids.

4. Solution de gouttes de confort de la revendication 1, dans laquelle ledit agent aplatissant est présent en une quantité de 0,15% à 0,3% en poids.

5. Solution de gouttes de confort de la revendication 1, dans laquelle ladite viscosité relative de Brookfield est dans un intervalle allant de 3 à 20 mPa.s (cps).

6. Solution de gouttes de confort de la revendication 5 dans laquelle ladite viscosité relative de Brookfield est dans un intervalle allant de 6 à 11 mPa.s (cps).

**7.** Solution de gouttes de confort de la revendication 1 dans laquelle ledit agent améliorant la viscosité est l'hydroéthylcellulose.

**8.** Solution de gouttes de confort de la revendication 1 dans laquelle ledit sel ionique est présent au moins en une quantité qui équivaut en osmolarité à une solution de chlorure de sodium à 0,3% en poids.

**9.** Solution de gouttes de confort de la revendication 1 dans laquelle ledit sel ionique est présent au moins en une quantité qui est équivalente en osmolarité à une solution de chlorure de sodium à 0,5% en poids.

**10.** Solution de gouttes de confort de la revendication 1, dans laquelle ledit sel ionique est présent au moins en une quantité qui équivaut en osmolarité à une solution de chlorure de sodium à 0,75%.

**11.** Solution de gouttes de confort de la revendication 1, dans laquelle ledit sel ionique est le chlorure de sodium.

**12.** Solution de gouttes de confort de la revendication 1, dans laquelle ledit agent de conservation est oculairement non irritant.

**13.** Solution de gouttes de confort de la revendication 1, dans laquelle ledit agent de conservation est présent en une quantité de 0,001 à 0,5% en poids desdites gouttes de confort.

**14.** Solution de gouttes de confort de la revendication 1, dans laquelle ledit agent de conservation est présent en une quantité de 0,1 à 0,25% en poids desdites gouttes de confort.

**15.** Solution de gouttes de confort de la revendication 1, dans laquelle ledit agent de conservation est l'acide sorbique.

**16.** Solution de gouttes de confort de la revendication 1, dans laquelle ledit pH desdites gouttes de confort est de 7,0 à 7,6.

**17.** Solution de gouttes de confort de la revendication 1, dans laquelle ledit pH desdites gouttes de confort est de 7,3.

**18.** Solution de gouttes de confort de la revendication 1, dans laquelle ledit tampon est un tampon au borate.

**19.** Solution de gouttes de confort de la revendication 1, dans laquelle ledit agent pharmaceutiquement actif oculairement administrable est un décongestionnant oculaire et est présent en une quantité à effet vasoconstrictif sur l'oeil.

**20.** Solution de gouttes de confort de la revendication 1 comprenant 0,2% en poids d'urée, 0,44% en poids de NaCl, 0,16% en poids d'acide borique, 0,38% en poids de tétraborate de sodium décahydraté ; 0,2% en poids d'EDTA disodique ; 0,15% en poids d'acide sorbique ; 0,7% en poids d'hydroxyéthyl-cellulose ; 0,2% en poids d'un copolymère oxyde d'éthylène-oxyde de propylène comme le Poloxamer® 407.

**21.** Solution de gouttes de confort ophtalmique pour utilisation intraoculaire avec lentilles de contact d'hydrogel pour utilisation prolongée afin d'améliorer l'échange
    I) Du fluide oculaire piégé entre ladite lentille et un oeil auquel ladite lentille a été appliquée pour
    II) Un fluide oculairement compatible non ainsi piégé, ladite solution comprenant :
        a) de 0,005 à 0,1% en poids d'un agent aplatissant de lentille choisi parmi l'urée, la glycérine, le sorbitol, l'aminoéthanol et leurs mélanges ;
        b) un sel ionique oculairement acceptable en une quantité suffisante pour donner à la totalité de la solution de gouttes de confort une tonicité située dans un intervalle de 270-330 milliosmoles/kg de ladite solution de gouttes de confort ;
        c) un agent améliorant la viscosité oculairement acceptable en une quantité suffisante pour donner à ladite solution de gouttes de confort une viscosité relative de Brookfield située dans un intervalle allant de 3 à 50 mPa.s (cps) ;

(d) un tampon oculairement acceptable en une quantité de 0% ou une quantité efficace comme tampon pour que ladite solution de gouttes de confort ait un pH de 6,5 à 8 ;

e) de 0% à 0,5% d'un agent de conservation oculairement acceptable ;

f) de 0% à 2% d'un lubrifiant oculairement acceptable ;

g) 0% ou une quantité pharmaceutiquement efficace et oculairement acceptable d'un agent pharmaceutiquement actif administrable par voie oculaire ; et

h) de 0% à 1% d'un agent tensio-actif oculairement acceptable non ionique.

**22.** Solution de gouttes de confort de la revendication 21, dans laquelle ledit agent aplatissant est l'urée.

**23.** Solution de gouttes de confort de la revendication 21, dans laquelle ledit agent aplatissant est présent en une quantité de 0,001% à 0,08% en poids.

**24.** Solution de gouttes de confort de la revendication 21, dans laquelle ledit agent aplatissant est présent en une quantité de 0,01% à 0,05% en poids.

**25.** Solution de gouttes de confort de la revendication 21, dans laquelle ladite viscosité relative de Brookfield est dans un intervalle de 3 à 20 mPa.s. (cps).

**26.** Solution de gouttes de confort de la revendication 25, dans laquelle viscosité relative de Brookfield est dans un intervalle allant de 6 à 11 mPa.s (cps).

**27.** Solution de gouttes de confort de la revendication 21, dans laquelle ledit agent améliorant la viscosité est l'hydroxyéthylcellulose.

**28.** Solution de gouttes de confort de la revendication 21, dans laquelle ledit sel ionique est présent au moins en une quantité qui est équivalente en osmolarité à une solution de chlorure de sodium à 0,3% en poids.

**29.** Solution de gouttes de confort de la revendication 21, dans laquelle ledit sel ionique est présent au moins en une quantité qui équivaut en osmolarité à une solution de chlorure de sodium à 0,5% en poids.

**30.** Solution de gouttes de confort de la revendication 21, dans laquelle ledit sel ionique est présent au moins en une quantité qui équivaut en osmolarité à une solution de chlorure de sodium à 0,75% en poids.

**31.** Solution de gouttes de confort de la revendication 21, dans laquelle ledit sel ionique est le chlorure de sodium.

**32.** Solution de gouttes de confort de la revendication 21, dans laquelle ledit agent de conservation est oculairement non irritant.

**33.** Solution de gouttes de confort de la revendication 21, dans laquelle ledit agent de conservation est présent en une quantité de 0,001 à 0,5% en poids desdites gouttes de confort.

**34.** Solution de gouttes de confort de la revendication 21, dans laquelle ledit agent de conservation est présent en une quantité de 0,1 à 0,25% en poids desdites gouttes de confort.

**35.** Solution de gouttes de confort de la revendication 21, dans laquelle ledit agent de conservation est l'acide sorbique.

**36.** Solution de gouttes de confort de la revendication 21, dans laquelle ledit pH desdites gouttes de confort est de 7,0 à 7,6.

**37.** Solution de gouttes de confort de la revendication 21, dans laquelle ledit pH desdites gouttes de confort est de 7,3.

**38.** Solution de gouttes de confort de la revendication 21, dans laquelle ledit tampon est un tampon au borate.

**39.** Solution de gouttes de confort de la revendication 21, dans laquelle ledit agent pharmaceutiquement actif, oculairement administrable est un décongestionnant oculaire et est présent en une quantité vasoconstrictrice efficace pour l'oeil.

**40.** Solution de gouttes de confort de la revendication 21, comprenant 0,03% en poids d'urée ; 0,52% en poids de NaCl ; 0,16% en poids d'acide borique ; 0,38% en poids de tétraborate de sodium décahydraté ; 0,2% en poids d'EDTA disodique ; 0,15% en poids d'acide sorbique ; 0,7% en poids d'hydroxyéthylcellulose ; 0,2% en poids d'un copolymère oxyde d'éthylène-oxyde de propylène comme le Poloxamer®407.

**41.** Application d'une solution de gouttes de confort comprenant :

a) de 0,1 à 0,5% en poids d'un agent aplatissant de lentille choisi parmi l'urée, la glycérine, le sorbitol, l'aminoéthanol et leurs mélanges ;

b) un sel ionique oculairement acceptable en une quantité suffisante pour donner à la totalité de la solution de gouttes de confort une tonicité située dans un intervalle de 270-330 milliosmoles/kg de ladite solution de gouttes de confort ;

c) un agent améliorant la viscosité oculairement acceptable en une quantité suffisante pour donner à ladite solution de gouttes de confort une viscosité relative de Brookfield située dans un intervalle allant de 3 à 50 mPa.s (cps) ;

d) un tampon oculairement acceptable en une quantité de 0% ou une quantité efficace comme tampon pour que ladite solution de gouttes de confort ait un pH de 6,5 à 8 ;

e) de 0% à 0,5% d'un agent de conservation oculairement acceptable ;

f) de 0% à 2% d'un lubrifiant oculairement acceptable ;

g) 0% ou une quantité pharmaceutiquement efficace et oculairement acceptable d'un agent pharmaceutiquement actif administrable par voie oculaire ; et

h) de 0% à 1% d'un agent tensio-actif oculairement acceptable non ionique. pour améliorer l'échange

I) du fluide situé entre une lentille de contact d'hydrogel à utilisation quotidienne et un oeil auquel ladite lentille a été appliquée, avec

II) un fluide oculairement compatible non ainsi situé, tandis que ladite lentille est portée sur ledit oeil dans laquelle on instille audit oeil, sur lequel se trouve ladite lentille, une quantité efficace pour améliorer les échanges de fluides de ladite solution de gouttes de confort, opération par laquelle ladite lentille est temporairement aplatie ce qui permet un plus grand échange de fluide que cela n'est possible en l'absence de ladite solution de gouttes de confort.

**42.** Application de la revendication 41, dans laquelle ladite solution de gouttes de confort est instillée en une quantité de 1 à 5 gouttes, jusqu'à cinq fois par jour.

**43.** Application d'une solution de gouttes de confort de la revendication 20 pour améliorer l'échange

I) du fluide situé entre une lentille de contact d'hydrogel à utilisation quotidienne et un oeil auquel ladite lentille a été appliquée avec

II) un fluide oculairement compatible non ainsi situé, tandis que ladite lentille est portée sur ledit oeil, dans laquelle on instille audit oeil, sur lequel se trouve ladite lentille, une quantité efficace pour améliorer les échanges de fluide de ladite solution de gouttes de confort de la revendication 1, opération

dans laquelle ladite lentille est temporairement aplatie, permettant un plus grand échange de fluide que cela n'est possible en l'absence de ladite solution de gouttes de confort de la revendication 20.

**44.** Application de la revendication 43, dans laquelle ladite solution de gouttes de confort est instillée en une quantité de 1 à 5 gouttes jusqu'à cinq fois par jour.

**45.** Application d'une solution de gouttes de confort de la revendication 21, dans laquelle le composant g) est absent pour améliorer l'échange

I) du fluide situé entre une lentille de contact d'hydrogel à utilisation prolongée et un oeil auquel ladite lentille a été appliquée, avec

23

II) un fluide oculairement compatible non ainsi localisé, tandis que ladite lentille est portée sur ledit oeil,

dans laquelle on instille audit oeil, sur lequel se trouve ladite lentille, une quantité efficace pour améliorer les échanges de fluide de ladite solution de gouttes de confort de la revendication 21, opération dans laquelle ladite lentille est temporairement aplatie, ce qui permet un plus grand échange de fluide que cela n'est possible en l'absence de ladite solution de gouttes de confort de la revendication 21.

**46.** Application de la revendication 45, dans laquelle ladite solution de gouttes de confort est instillée en une quantité de 1 à 5 gouttes jusqu'à cinq fois par jour.

**47.** Application d'une solution de gouttes de confort de la revendication 40 pour améliorer l'échange
I) du fluide situé entre une lentille de contact d'hydrogel à utilisation prolongée et un oeil auquel ladite lentille a été appliquée, avec
II) un fluide oculairement compatible non ainsi situé, tandis que ladite lentille est portée sur ledit oeil
dans laquelle on instille audit oeil, sur lequel se trouve la lentille, une quantité efficace pour améliorer les échanges de fluide de ladite solution de gouttes de confort de la revendication 40, opération dans laquelle ladite lentille est temporairement aplatie, permettant un plus grand échange de fluide que cela n'est possible en l'absence de ladite solution de gouttes de confort de la revendication 40.

**48.** Application de la revendication 47 dans laquelle ladite solution de gouttes de confort est instillée en une quantité de 1 à 5 gouttes jusqu'à cinq fois par jour.

**Revendications pour l'Etat contractant suivant : AT**

**1.** Procédé de préparation d'une solution ophtalmique de gouttes de confort pour utilisation intra-oculaire avec une lentille de contact d'hydrogel à porter quotidiennement pour améliorer l'échange
I) Du fluide oculaire piégé entre ladite lentille et un oeil auquel ladite lentille a été appliquée pour
II) Un fluide oculairement compatible non ainsi piégé, ladite solution comprenant :
a) de 0,1 à 0,5% en poids d'un agent aplatissant de lentille choisi parmi l'urée, la glycérine, le sorbitol, l'aminoéthanol et leurs mélanges ; à condition que la quantité de glycérine soit inférieure à 0,4% en poids ;
b) un sel ionique oculairement acceptable en une quantité suffisante pour donner à la totalité de la solution de gouttes de confort une tonicité située dans un intervalle de 270-330 milliosmoles/kg de ladite solution de gouttes de confort ;
c) un agent améliorant la viscosité oculairement acceptable en une quantité suffisante pour donner à ladite solution de gouttes de confort une viscosité relative de Brookfield située dans un intervalle allant de 3 à 50 mPa.s (cps) ;
(d) un tampon oculairement acceptable en une quantité de 0% ou une quantité efficace comme tampon pour que ladite solution de gouttes de confort ait un pH de 6,5 à 8 ;
e) de 0% à 0,5% d'un agent de conservation oculairement acceptable ;
f) de 0% à 2% d'un lubrifiant oculairement acceptable ;
g) 0% ou une quantité pharmaceutiquement efficace et oculairement acceptable d'un agent pharmaceutiquement actif administrable par voie oculaire ; et
h) de 0% à 1% d'un agent tensio-actif oculairement acceptable non ionique ; par mélange des composants de façon classique.

**2.** Procédé de la revendication 1, dans lequel ledit agent aplatissant est l'urée.

**3.** Procédé de la revendication 1, dans lequel ledit agent aplatissant est présent en une quantité de 0,1% à 0,4% en poids.

**4.** Procédé de la revendication 1, dans lequel ledit agent aplatissant est présent en une quantité de 0,15% à 0,3% en poids.

**5.** Procédé de la revendication 1, dans lequel ladite viscosité relative de Brookfield est dans un intervalle allant de 3 à 20 mPa.s (cps).

24

**6.** Procédé de la revendication 5 dans lequel ladite viscosité relative de Brookfield est dans un intervalle allant de 6 à 11 mPa.s (cps).

**7.** Procédé de la revendication 1 dans lequel ledit agent améliorant la viscosité est l'hydroéthylcellulose.

**8.** Procédé de la revendication 1 dans lequel ledit sel ionique est présent au moins en une quantité qui équivaut en osmolarité à une solution de chlorure de sodium à 0,3% en poids.

**9.** Procédé de la revendication 1 dans lequel ledit sel ionique est présent au moins en une quantité qui est équivalente en osmolarité à une solution de chlorure de sodium à 0,5% en poids.

**10.** Procédé de la revendication 1, dans lequel ledit sel ionique est présent au moins en une quantité qui équivaut en osmolarité à une solution de chlorure de sodium à 0,75%.

**11.** Procédé de la revendication 1, dans lequel ledit sel ionique est le chlorure de sodium.

**12.** Procédé de la revendication 1, dans lequel ledit agent de conservation est oculairement non irritant.

**13.** Procédé de la revendication 1, dans lequel ledit agent de conservation est présent en une quantité de 0,001 à 0,5% en poids desdites gouttes de confort.

**14.** Procédé de la revendication 1, dans lequel ledit agent de conservation est présent en une quantité de 0,1 à 0,25% en poids desdites gouttes de confort.

**15.** Procédé de la revendication 1, dans lequel ledit agent de conservation est l'acide sorbique.

**16.** Procédé de la revendication 1, dans lequel ledit pH desdites gouttes de confort est de 7,0 à 7,6.

**17.** Procédé de la revendication 1, dans lequel ledit pH desdites gouttes de confort est de 7,3.

**18.** Procédé de la revendication 1, dans lequel ledit tampon est un tampon au borate.

**19.** Procédé de la revendication 1, dans lequel ledit agent pharmaceutiquement actif oculairement administrable est un décongestionnant oculaire et est présent en une quantité à effet vasoconstrictif sur l'oeil.

**20.** Procédé de la revendication 1 comprenant 0,2% en poids d'urée, 0,44% en poids de NaCl, 0,16% en poids d'acide borique, 0,38% en poids de tétraborate de sodium décahydraté ; 0,2% en poids d'EDTA disodique ; 0,15% en poids d'acide sorbique ; 0,7% en poids d'hydroxyéthylcellulose ; 0,2% en poids d'un copolymère oxyde d'éthylène-oxyde de propylène comme le Poloxamer^407.

**21.** Procédé de préparation d'une solution de gouttes de confort ophtalmique pour utilisation intraoculaire avec lentilles de contact d'hydrogel pour utilisation prolongée afin d'améliorer l'échange
    I) Du fluide oculaire piégé entre ladite lentille et un oeil auquel ladite lentille a été appliquée pour
    II) Un fluide oculairement compatible non ainsi piégé, ladite solution comprenant :
        a) de 0,0005 à 0,1% en poids d'un agent aplatissant de lentille choisi parmi l'urée, la glycérine, le sorbitol, l'aminoéthanol et leurs mélanges ;
        b) un sel ionique oculairement acceptable en une quantité suffisante pour donner à la totalité de la solution de gouttes de confort une tonicité située dans un intervalle de 270-330 milliosmoles/kg de ladite solution de gouttes de confort ;
        c) un agent améliorant la viscosité oculairement acceptable en une quantité suffisante pour donner à ladite solution de gouttes de confort une viscosité relative de Brookfield située dans un intervalle allant de 3 à 50 mPa.s (cps) ;
        (d) un tampon oculairement acceptable en une quantité de 0% ou une quantité efficace comme tampon pour que ladite solution de gouttes de confort ait un pH de 6,5 à 8 ;
        e) de 0% à 0,5% d'un agent de conservation oculairement acceptable ;
        f) de 0% à 2% d'un lubrifiant oculairement acceptable ;
        g) 0% ou une quantité pharmaceutiquement efficace et oculairement acceptable d'un agent pharmaceutiquement actif administrable par voie oculaire ; et

h) de 0% à 1% d'un agent tensio-actif oculairement acceptable non ionique ;
par mélange des composants de façon classique.

22. Procédé de la revendication 21, dans lequel ledit agent aplatissant est l'urée.

23. Procédé de la revendication 21, dans lequel ledit agent aplatissant est présent en une quantité de 0,001% à 0,08% en poids.

24. Procédé de la revendication 21, dans lequel ledit agent aplatissant est présent en une quantité de 0,01% à 0,05% en poids.

25. Procédé de la revendication 21, dans lequel ladite viscosité relative de Brookfield est dans un intervalle de 3 à 20 mPa.s. (cps).

26. Procédé de la revendication 25, dans lequel viscosité relative de Brookfield est dans un intervalle allant de 6 à 11 mPa.s (cps).

27. Procédé de la revendication 21, dans lequel ledit agent améliorant la viscosité est l'hydroxyéthylcellulose.

28. Procédé de la revendication 21, dans lequel ledit sel ionique est présent au moins en une quantité qui est équivalente en osmolarité à une solution de chlorure de sodium à 0,3% en poids.

29. Procédé de la revendication 21, dans lequel ledit sel ionique est présent au moins en une quantité qui équivaut en osmolarité à une solution de chlorure de sodium à 0,5% en poids.

30. Procédéde la revendication 21, dans lequel ledit sel ionique est présent au moins en une quantité qui équivaut en osmolarité à une solution de chlorure de sodium à 0,75% en poids.

31. Procédé de la revendication 21, dans lequel ledit sel ionique est le chlorure de sodium.

32. Procédé de la revendication 21, dans lequel ledit agent de conservation est oculairement non irritant.

33. Procédé de la revendication 21, dans lequel ledit agent de conservation est présent en une quantité de 0,001 à 0,5% en poids desdites gouttes de confort.

34. Procédé de la revendication 21, dans lequel ledit agent de conservation est présent en une quantité de 0,1 à 0,25% en poids desdites gouttes de confort.

35. Procédé de la revendication 21, dans lequel ledit agent de conservation est l'acide sorbique.

36. Procédé de la revendication 21, dans lequel ledit pH desdites gouttes de confort est de 7,0 à 7,6.

37. Procédé de la revendication 21, dans lequel ledit pH desdites gouttes de confort est de 7,3.

38. Procédé de la revendication 21, dans lequel ledit tampon est un tampon au borate.

39. • Procédé de la revendication 21, dans lequel ledit agent pharmaceutiquement actif, oculairement administrable est un décongestionnant oculaire et est présent en une quantité vasoconstrictrice efficace pour l'oeil.

40. Procédé de la revendication 21, dans lequel ladite solution de gouttes de confort est une solution comprenant 0,03% en poids d'urée ; 0,52% en poids de NaCl ; 0,16% en poids d'acide borique ; 0,38% en poids de tétraborate de sodium décahydraté ; 0,2% en poids d'EDTA disodique ; 0,15% en poids d'acide sorbique ; 0,7% en poids d'hydroxyéthylcellulose ; 0,2% en poids d'un copolymère oxyde d'éthylène-oxyde de propylène comme le Poloxamer ^ 407.

41. Application d'une solution de gouttes de confort comprenant :

a) de 0,1 à 0,5% en poids d'un agent aplatissant de lentille choisi parmi l'urée, la glycérine, le sorbitol, l'aminoéthanol et leurs mélanges ;

b) un sel ionique oculairement acceptable en une quantité suffisante pour donner à la totalité de la solution de gouttes de confort une tonicité située dans un intervalle de 270-330 milliosmoles/kg de ladite solution de gouttes de confort ;

c) un agent améliorant la viscosité oculairement acceptable en une quantité suffisante pour donner à ladite solution de gouttes de confort une viscosité relative de Brookfield située dans un intervalle allant de 3 à 50 mPa.s (cps) ;

d) un tampon oculairement acceptable en une quantité de 0% ou une quantité efficace comme tampon pour que ladite solution de gouttes de confort ait un pH de 6,5 à 8 ;

e) de 0% à 0,5% d'un agent de conservation oculairement acceptable ;

f) de 0% à 2% d'un lubrifiant oculairement acceptable ;

g) 0% ou une quantité pharmaceutiquement efficace et oculairement acceptable d'un agent pharmaceutiquement actif administrable par voie oculaire ; et

h) de 0% à 1% d'un agent tensio-actif oculairement acceptable non ionique. pour améliorer l'échange

I) du fluide situé entre une lentille de contact d'hydrogel à utilisation quotidienne et un oeil auquel ladite lentille a été appliquée, avec

II) un fluide oculairement compatible non ainsi situé, tandis que ladite lentille est portée sur ledit oeil dans laquelle on instille audit oeil, sur lequel se trouve ladite lentille, une quantité efficace pour améliorer les échanges de fluides de ladite solution de gouttes de confort, opération par laquelle ladite lentille est temporairement aplatie ce qui permet un plus grand échange de fluide que cela n'est possible en l'absence de ladite solution de gouttes de confort.

42. Application de la revendication 41, dans laquelle ladite solution de gouttes de confort est instillée en une quantité de 1 à 5 gouttes, jusqu'à cinq fois par jour.

43. Application d'une solution de gouttes de confort de la revendication 20 pour améliorer l'échange

I) du fluide situé entre une lentille de contact d'hydrogel à utilisation quotidienne et un oeil auquel ladite lentille a été appliquée avec

II) un fluide oculairement compatible non ainsi situé, tandis que ladite lentille est portée sur ledit oeil, dans laquelle on instille audit oeil, sur lequel se trouve ladite lentille, une quantité efficace pour améliorer les échanges de fluide de ladite solution de gouttes de confort de la revendication 1, opération dans laquelle ladite lentille est temporairement aplatie, permettant un plus grand échange de fluide que cela n'est possible en l'absence de ladite solution de gouttes de confort de la revendication 20.

44. Application de la revendication 43, dans laquelle ladite solution de gouttes de confort est instillée en une quantité de 1 à 5 gouttes jusqu'à cinq fois par jour.

45. Application d'une solution de gouttes de confort de la revendication 21, dans laquelle le composant g) est absent pour améliorer l'échange

I) du fluide situé entre une lentille de contact d'hydrogel à utilisation prolongée et un oeil auquel ladite lentille a été appliquée, avec

II) un fluide oculairement compatible non ainsi localisé, tandis que ladite lentille est portée sur ledit oeil,

dans laquelle on instille audit oeil, sur lequel se trouve ladite lentille, une quantité efficace pour améliorer les échanges de fluide de ladite solution de gouttes de confort de la revendication 21, opération dans laquelle ladite lentille est temporairement aplatie, ce qui permet un plus grand échange de fluide que cela n'est possible en l'absence de ladite solution de gouttes de confort de la revendication 21.

46. Application de la revendication 45, dans laquelle ladite solution de gouttes de confort est instillée en une quantité de 1 à 5 gouttes jusqu'à cinq fois par jour.

47. Application d'une solution de gouttes de confort de la revendication 40 pour améliorer l'échange

I) du fluide situé entre une lentille de contact d'hydrogel à utilisation prolongée et un oeil auquel ladite lentille a été appliquée, avec

II) un fluide oculairement compatible non ainsi situé, tandis que ladite lentille est portée sur ledit oeil dans laquelle on instille audit oeil, sur lequel se trouve la lentille, une quantité efficace pour améliorer les échanges de fluide de ladite solution de gouttes de confort de la revendication 40, opération dans laquelle ladite lentille est temporairement aplatie, permettant un plus grand échange de fluide que cela n'est possible en l'absence de ladite solution de gouttes de confort de la revendication 40.

48. Application de la revendication 47 dans laquelle ladite solution de gouttes de confort est instillée en une quantité de 1 à 5 gouttes jusqu'à cinq fois par jour.

**Patentansprüche**
**Patentansprüche für folgende Verstragsstaaten : BE, CH, DE, FR, GB, IT, LI, LU, NL, SE**

1. Ophthalmische Komfort-Tropflösung für intraokulare Verwendung bei einer tagsüber zu tragenden Hydrogel-Kontaktlinse zur Verbesserung des Austauschs von I) okularem Fluid, das zwischen der Linse und einem Auge, in dem sich die Linse befindet, eingeschlossen ist, gegen II) mit dem Auge verträgliches Fluid, das nicht so eingeschlossen ist, welche Lösung umfaßt:
a) 0,1 bis 0,5 Gew.-% eines Linsenabflachungsmittels, das ausgewählt wird aus der Gruppe Harnstoff, Glycerin, Sorbit, Aminoethanol und deren Gemische, vorausgesetzt, daß die Glycerinmenge unter 0,4 Gew.-% beträgt; b) ein für das Auge verträgliches ionisches Salz in einer Menge, die ausreichend ist, der gesamten Komfort-Tropflösung eine Tonizität im Bereich von 270 bis 330 milliosmol pro Kilogramm der genannten Komfort-Tropflösung zu verleihen; c) ein für das Auge verträgliches die Viskosität erhöhendes Mittel in ausreichender Menge, um der Komfort-Tropflösung eine relative Brookfield-Viskosität im Bereich von 3 bis 50 mPa.s (cps) zu verleihen; d) einen für das Auge verträglichen Puffer in einer Menge von 0 % oder einer wirksamen puffemden Menge, um zu einer Komfort-Tropflösung mit einem pH-Wert von 6,5 bis 8 zu führen; e) 0 % bis 0,5 % eines für das Auge verträglichen Konservierungsmittels; f) 0 % bis 2 % eines für das Auge verträglichen Gleitmittels; g) 0 % oder eine für das Auge annehmbare pharmazeutisch wirksame Menge eines okular verabreichbaren pharmazeutisch aktiven Mittels; und h) 0 % bis 1 % eines nichtionischen für das Auge annehmbaren oberflächenaktiven Mittels.

2. Komfort-Tropflösung nach Anspruch 1, worin das genannte Abflachungsmittel Harnstoff ist.

3. Komfort-Tropflösung nach Anspruch 1, worin das genannte Abflachungsmittel in einer Menge von 0,1 bis 0,4 Gew.-% vorliegt.

4. Komfort-Tropflösung nach Anspruch 1, worin das genannte Abflachungsmittel in einer Menge von 0,15 bis 0,3 Gew.-% vorliegt.

5. Komfort-Tropflösung nach Anspruch 1, worin die genannte relative Brookfield-Viskosität im Bereich von 3 bis 20 mPa.s (cps) liegt.

6. Komfort-Tropflösung nach Anspruch 5, worin die genannte relative Brookfield-Viskosität im Bereich von 6 bis 11 mPa.s (cps) liegt.

7. Komfort-Tropflösung nach Anspruch 1, worin das genannte die Viskosität erhöhende Mittel Hydroxyethylcellulose ist.

8. Komfort-Tropflösung nach Anspruch 1, worin das genannte ionische Salz in zumindest einer Menge vorhanden ist, die hinsichtlich der Osmolarität einer Lösung von 0,3 Gew.-% Natriumchlorid äquivalent ist.

9. Komfort-Tropflösung nach Anspruch 1, worin das genannte ionische Salz in zumindest einer Menge vorhanden ist, die hinsichtlich der Osmolarität einer Lösung von 0,5 Gew.-% Natriumchlorid äquivalent ist.

10. Komfort-Tropflösung nach Anspruch 1, worin das genannte ionische Salz in zumindest einer Menge vorhanden ist, die hinsichtlich der Osmolarität einer Lösung von 0,75 Gew.-% Natriumchlorid äquivalent ist.

**11.** Komfort-Tropflösung nach Anspruch 1, worin das genannte ionische Salz Natriumchlorid ist

**12.** Komfort-Tropflösung nach Anspruch 1, worin das genannte Konservierungsmittel das Auge nicht irritiert.

**13.** Komfort-Tropflösung nach Anspruch 1, worin das genannte Konservierungsmittel in einer Menge von 0,001 bis 0,5 Gew.-% der Komfort-Tropfen vorliegt.

**14.** Komfort-Tropflösung nach Anspruch 1, worin das genannte Konservierungsmittel in einer Menge von 0,1 bis 0,25 Gew.-% der Komfort-Tropfen vorliegt.

**15.** Komfort-Tropflösung nach Anspruch 1, worin das genannte Konservierungsmittel Sorbinsäure ist.

**16.** Komfort-Tropflösung nach Anspruch 1, worin der pH-Wert der Komfort-Tropfen 7,0 bis 7,6 beträgt.

**17.** Komfort-Tropflösung nach Anspruch 1, worin der pH-Wert der Komfort-Tropfen 7,3 beträgt.

**18.** Komfort-Tropflösung nach Anspruch 1, worin der genannte Puffer ein Boratpuffer ist.

**19.** Komfort-Tropflösung nach Anspruch 1, worin das okular verabreichbare pharmazeutisch aktive Mittel ein Augen-Dekongestionsmittel und in einer okular gefäßverengend wirksamen Menge vorhanden ist.

**20.** Komfort-Tropflösung nach Anspruch 1, umfassend 0,2 Gew.-% Harnstoff; 0,44 Gew.-% NaCl; 0,16 Gew.-% Borsäure; 0,38 Gew.-% Na-Tetraborat-decahydrat; 0,2 Gew.-% Dinatrium-EDTA; 0,15 Gew.-% Sorbinsäure; 0,7 Gew.-% Hydroxyethylcellulose; 0,2 Gew.-% eines Ethylenoxid-Propylenoxid-Copolymers wie Poloxamer$^{(R)}$407.

**21.** Ophthalmische Komfort-Tropflösung für intraokulare Verwendung bei einer Hydrogel-Kontaktlinse für verlängerte Tragedauer zur Verbesserung des Austauschs von I) okularem Fluid, das zwischen der Linse und einem Auge, in dem sich die Linse befindet, eingeschlossen ist, gegen II) mit dem Auge verträgliches Fluid, das nicht so eingeschlossen ist, welche Lösung umfaßt:
a) 0,0005 bis 0,1 Gew.-% eines Linsenabflachungsmittels, das ausgewählt wird aus der Gruppe Harnstoff, Glycerin, Sorbit, Aminoethanol und deren Gemische; b) ein für das Auge verträgliches ionisches Salz in einer Menge, die ausreichend ist, der gesamten Komfort-Tropflösung eine Tonizität im Bereich von 270 bis 330 milliosmol pro Kilogramm der genannten Komfort-Tropflösung zu verleihen; c) ein für das Auge verträgliches die Viskosität erhöhendes Mittel in ausreichender Menge, um der Komfort-Tropflösung eine relative Brookfield-Viskosität im Bereich von 3 bis 50 mPa.s (cps) zu verleihen; d) einen für das Auge verträglichen Puffer in einer Menge von 0 % oder einer wirksamen puffernden Menge, um zu einer Komfort-Tropflösung mit einem pH-Wert von 6,5 bis 8 zu führen; e) 0 % bis 0,5 % eines für das Auge verträglichen Konservierungsmittels; f) 0 % bis 2 % eines für das Auge verträglichen Gleitmittels; g) 0 % oder eine für das Auge annehmbare pharmazeutisch wirksame Menge eines okular verabreichbaren pharmazeutisch aktiven Mittels; und h) 0 % bis 1 % eines nichtionischen für das Auge annehmbaren oberflächenaktiven Mittels.

**22.** Komfort-Tropflösung nach Anspruch 21, worin das genannte Abflachungsmittel Harnstoff ist.

**23.** Komfort-Tropflösung nach Anspruch 21, worin das genannte Abflachungsmittel in einer Menge von 0,001 bis 0,08 Gew.-% vorliegt.

**24.** Komfort-Tropflösung nach Anspruch 21, worin das genannte Abflachungsmittel in einer Menge von 0,01 bis 0,05 Gew.-% vorliegt.

**25.** Komfort-Tropflösung nach Anspruch 21, worin die genannte relative Brookfield-Viskosität im Bereich von 3 bis 20 mPa.s (cps) liegt.

**26.** Komfort-Tropflösung nach Anspruch 25, worin die genannte relative Brookfield-Viskosität im Bereich von 6 bis 11 mPa.s (cps) liegt.

**27.** Komfort-Tropflösung nach Anspruch 21, worin das genannte die Viskosität erhöhende Mittel Hydroxyethylcellulose ist.

**28.** Komfort-Tropflösung nach Anspruch 21, worin das genannte ionische Salz in zumindest einer Menge vorhanden ist, die hinsichtlich der Osmolarität einer Lösung von 0,3 Gew.-% Natriumchlorid äquivalent ist.

**29.** Komfort-Tropflösung nach Anspruch 21, worin das genannte ionische Salz in zumindest einer Menge vorhanden ist, die hinsichtlich der Osmolarität einer Lösung von 0,5 Gew.-% Natriumchlorid äquivalent ist.

**30.** Komfort-Tropflösung nach Anspruch 21, worin das genannte ionische Salz in zumindest einer Menge vorhanden ist, die hinsichtlich der Osmolarität einer Lösung von 0,75 Gew.-% Natriumchlorid äquivalent ist.

**31.** Komfort-Tropflösung nach Anspruch 21, worin das genannte ionische Salz Natriumchlorid ist.

**32.** Komfort-Tropflösung nach Anspruch 21, worin das genannte Konservierungsmittel das Auge nicht irritiert.

**33.** Komfort-Tropflösung nach Anspruch 21, worin das genannte Konservierungsmittel in einer Menge von 0,001 bis 0,5 Gew.-% der Komfort-Tropfen vorliegt.

**34.** Komfort-Tropflösung nach Anspruch 21, worin das genannte Konservierungsmittel in einer Menge von 0,1 bis 0,25 Gew.-% der Komfort-Tropfen vorliegt.

**35.** Komfort-Tropflösung nach Anspruch 21, worin das genannte Konservierungsmittel Sorbinsäure ist.

**36.** Komfort-Tropflösung nach Anspruch 21, worin der pH-Wert der Komfort-Tropfen 7,0 bis 7,6 beträgt.

**37.** Komfort-Tropflösung nach Anspruch 21, worin der pH-Wert der Komfort-Tropfen 7,3 beträgt.

**38.** Komfort-Tropflösung nach Anspruch 21, worin der genannte Puffer ein Boratpuffer ist.

**39.** Komfort-Tropflösung nach Anspruch 21, worin das okular verabreichbare pharmazeutisch aktive Mittel ein Augen-Dekongestionsmittel und in einer okular gefäßverengend wirksamen Menge vorhanden ist.

**40.** Komfort-Tropflösung nach Anspruch 21, umfassend 0,03 Gew.-% Harnstoff; 0,52 Gew.-% NaCl; 0,16 Gew.-% Borsäure; 0,38 Gew.-% Na-Tetraborat-decahydrat; 0,2 Gew.-% Dinatrium-EDTA; 0,15 Gew.-% Sorbinsäure; 0,7 Gew.-% Hydroxyethylcellulose; 0,2 Gew.-% eines Ethylenoxid-Propylenoxid-Copolymers wie Poloxamer[R]407.

**41.** Verwendung einer Komfort-Tropflösung, umfassend:
a) 0,1 bis 0,5 Gew.-% eines Linsenabflachungsmittels, das ausgewählt wird aus der Gruppe Harnstoff, Glycerin, Sorbit, Aminoethanol und deren Gemische; b) ein für das Auge veträgliches ionisches Salz in einer Menge, die ausreichend ist, der gesamten Komfort-Tropflösung eine Tonizität im Bereich von 270 bis 330 milliosmol pro Kilogramm der genannten Komfort-Tropflösung zu verleihen; c) ein für das Auge verträgliches die Viskosität erhöhendes Mittel in ausreichender Menge, um der Komfort-Tropflösung eine relative Brookfield-Viskosität im Bereich von 3 bis 50 mPa.s (cps) zu verleihen; d) einen für das Auge verträglichen Puffer in einer Menge von 0 % oder einer wirksamen puffernden Menge, um zu einer Komfort-Tropflösung mit einem pH-Wert von 6,5 bis 8 zu führen; e) 0 % bis 0,5 % eines für das Auge verträglichen Konservierungsmittels; f) 0 % bis 2 % eines für das Auge verträglichen Gleitmittels; g) 0 % eines okular verabreichbaren pharmazeutisch aktiven Mittels; und h) 0 % bis 1 % eines nichtionischen für das Auge annehmbaren oberflächenaktiven Mittels, zur Verbesserung des Austauschs von I) Fluid, das sich zwischen einer tagsüber zu tragenden Hydrogel-Kontaktlinse und einem Auge, auf das die Linse aufgebracht wurde, befindet, gegen II) ein mit dem Auge verträgliches Fluid, das nicht so lokalisiert ist, während die Linse im Auge getragen wird,
umfassend das Eintropfen einer den Fluidaustausch verbessernden wirksamen Menge der genannten Komfort-Tropflösung in das Auge, in dem sich die Linse befindet, wobei diese Linse temporär abgeflacht wird, was größeren Fluidaustausch ermöglicht als in Abwesenheit der genannten Komfort-Tropflösung möglich ist.

42. Verwendung nach Anspruch 41, worin die genannte Komfort-Tropflösung in einer Menge von 1 bis 5 Tropfen bis zu 5 Mal am Tag eingetropft wird.

43. Verwendung der Komfort-Tropflösung nach Anspruch 20 zur Verbesserung des Austauschs von I) Fluid, das sich zwischen einer tagsüber zu tragenden Hydrogel-Kontaktlinse und einem Auge, auf das die Linse aufgebracht wurde, befindet, gegen II) mit dem Auge verträgliches Fluid, das nicht so lokalisiert ist, wahrend die Linse im Auge getragen wird, umfassend das Eintropfen einer den Fluidaustausch verbessernden wirksamen Menge der genannten Komfort-Tropflösung nach Anspruch 20 in das Auge, in dem sich die Linse befindet, wobei diese Linse temporär abgeflacht wird, was größeren Fluidaustausch ermöglicht als in Abwesenheit der genannten Komfort-Tropflösung nach Anspruch 20 möglich ist.

44. Verwendung nach Anspruch 43, worin die genannte Komfort-Tropflösung in einer Menge von 1 bis 5 Tropfen bis zu 5 Mal am Tag eingetropft wird.

45. Verwendung einer Komfort-Tropflösung nach Anspruch 21, worin die Komponente g) abwesend ist, zur Verbesserung des Austauschs von I) Fluid, das sich zwischen einer Hydrogel-Dauertragelinse und einem Auge, auf das die Linse aufgebracht ist, befindet, gegen II) mit dem Auge verträgliches Fluid, das nicht so lokalisiert ist, während die Linse im Auge getragen wird, umfassend das Eintropfen einer den Fluidaustausch verbessernden wirksamen Menge der genannten Komfort-Tropflösung nach Anspruch 21 in das Auge, in dem sich die Linse befindet, wobei diese Linse temporär abgeflacht wird, was größeren Fluidaustausch ermöglicht als in Abwesenheit der genannten Komfort-Tropflösung nach Anspruch 21 möglich ist.

46. Verwendung nach Anspruch 45, worin die genannte Komfort-Tropflösung in einer Menge von 1 bis 5 Tropfen bis zu 5 Mal am Tag eingetropft wird.

47. Verwendung einer Komfort-Tropflösung nach Anspruch 40 zur Verbesserung des Austauschs von I) Fluid, das sich zwischen einer Hydrogel-Dauertragelinse und einem Auge, auf das die Linse aufgebracht ist, befindet, gegen II) mit dem Auge verträgliches Fluid, das nicht so lokalisiert ist, während die Linse im Auge getragen wird,
umfassend das Eintropfen einer den Fluidaustausch verbessernden wirksamen Menge der genannten Komfort-Tropflösung nach Anspruch 40 in das Auge, in dem sich die Linse befindet, wobei diese Linse temporär abgeflacht wird, was größeren Fluidaustausch ermöglicht als in Abwesenheit der genannten Komfort-Tropflösung nach Anspruch 40 möglich ist.

48. Verwendung nach Anspruch 47, worin die genannte Komfort-Tropflösung in einer Menge von 1 bis 5 Tropfen bis zu 5 Mal am Tag eingetropft wird.

**Patentansprüche für folgende Vertragsstraaten : AT**

1. Verfahren zur Herstellung einer ophthalmischen Komfort-Tropflösung für intraokulare Verwendung bei einer tagsüber zu tragenden Hydrogel-Kontaktlinse zur Verbesserung des Austauschs von I) okularem Fluid, das zwischen der Linse und einem Auge, in dem sich die Linse befindet, eingeschlossen ist, gegen II) mit dem Auge verträgliches Fluid, das nicht so eingeschlossen ist, welche Lösung umfaßt:
a) 0,1 bis 0,5 Gew.-% eines Linsenabflachungsmittels, das ausgewählt wird aus der Gruppe Hamstoff; Glycerin, Sorbit, Aminoethanol und deren Gemische; vorausgesetzt, daß die Glycerinmenge unter 0,4 Gew.-% beträgt; b) ein für das Auge verträgliches ionisches Salz in einer Menge, die ausreichend ist, der gesamten Komfort-Tropflösung eine Tonizität im Bereich von 270 bis 330 milliosmol pro Kilogramm der genannten Komfort-Tropflösung zu verleihen; c) ein für das Auge verträgliches die Viskosität erhöhendes Mittel in ausreichender Menge, um der Komfort-Tropflösung eine relative Brookfield-Viskosität im Bereich von 3 bis 50 mPa.s (cps) zu verleihen; d) einen für das Auge verträglichen Puffer

EP 0 198 490 B1

in einer Menge von 0 % oder einer wirksamen puffernden Menge, um zu einer Komfort-Tropflösung mit einem pH-Wert von 6,5 bis 8 zu führen; e) 0 % bis 0,5 % eines für das Auge verträglichen Konservierungsmittels; f) 0 % bis 2 % eines für das Auge verträglichen Gleitmittels; g) 0 % oder eine für das Auge annehmbare pharmazeutisch wirksame Menge eines okular verabreichbaren pharmazeutisch aktiven Mittels; und h) 0 % bis 1 % eines nichtionischen für das Auge annehmbaren oberflächenaktiven Mittels; durch herkömmliches Mischen der Komponenten.

2. Verfahren nach Anspruch 1, worin das genannte Abflachungsmittel Harnstoff ist.

3. Verfahren nach Anspruch 1, worin das genannte Abflachungsmittel in einer Menge von 0,1 bis 0,4 Gew.-% vorliegt.

4. Verfahren nach Anspruch 1, worin das genannte Abflachungsmittel in einer Menge von 0,15 bis 0,3 Gew.-% vorliegt.

5. Verfahren nach Anspruch 1, worin die genannte relative Brookfield-Viskosität im Bereich von 3 bis 20 mPa.s (cps) liegt.

6. Verfahren nach Anspruch 5, worin die genannte relative Brookfield-Viskosität im Bereich von 6 bis 11 mPa.s (cps) liegt.

7. Verfahren nach Anspruch 1, worin das genannte die Viskosität erhöhende Mittel Hydroxyethylcellulose ist.

8. Verfahren nach Anspruch 1, worin das genannte ionische Salz in zumindest einer Menge vorhanden ist, die hinsichtlich der Osmolarität einer Lösung von 0,3 Gew.-% Natriumchlorid äquivalent ist.

9. Verfahren nach Anspruch 1, worin das genannte ionische Salz in zumindest einer Menge vorhanden ist, die hinsichtlich der Osmolarität einer Lösung von 0,5 Gew.-% Natriumchlorid äquivalent ist.

10. Verfahren nach Anspruch 1, worin das genannte ionische Salz in zumindest einer Menge vorhanden ist, die hinsichtlich der Osmolarität einer Lösung von 0,75 Gew.-% Natriumchlorid äquivalent ist.

11. Verfahren nach Anspruch 1, worin das genannte ionische Salz Natriumchlorid ist.

12. Verfahren nach Anspruch 1, worin das genannte Konservierungsmittel das Auge nicht irritiert

13. Verfahren nach Anspruch 1, worin das genannte Konservierungsmittel in einer Menge von 0,001 bis 0,5 Gew.-% der Komfort-Tropfen vorliegt.

14. Verfahren nach Anspruch 1, worin das genannte Konservierungsmittel in einer Menge von 0,1 bis 0,25 Gew.-% der Komfort-Tropfen vorliegt.

15. Verfahren nach Anspruch 1, worin das genannte Konservierungsmittel Sorbinsäure ist

16. Verfahren nach Anspruch 1, worin der pH-Wert der Komfort-Tropfen 7,0 bis 7,6 beträgt.

17. Verfahren nach Anspruch 1, worin der pH-Wert der Komfort-Tropfen 7,3 beträgt.

18. Verfahren nach Anspruch 1, worin der genannte Puffer ein Boratpuffer ist.

19. Verfahren nach Anspruch 1, worin das okular verabreichbare pharmazeutisch aktive Mittel ein Augen-Dekongestionsmittel und in einer okular gefäßverengend wirksamen Menge vorhanden ist.

20. Verfahren nach Anspruch 1, worin die genannte Komfort-Tropflösung 0,2 Gew.-% Hamstoff; 0,44 Gew.-% NaCl; 0,16 Gew.-% Borsäure; 0,38 Gew.-% Na-Tetraborat-decahydrat; 0,2 Gew.-% Dinatrium-EDTA; 0,15 Gew.-% Sorbinsäure; 0,7 Gew.-% Hydroxyethylcellulose; 0,2 Gew.-% eines Ethylenoxid-Propylenoxid-Copolymers wie Poloxamer[R]407 enthält.

32

**21.** Verfahren zur Herstellung einer ophthalmischen Komfort-Tropflösung für intraokulare Verwendung bei einer Hydrogel-Dauertragelinse zur Verbesserung des Austauschs von I) okularem Fluid, das zwischen der Linse und einem Auge, in dem sie sich befindet, eingeschlossen ist, gegen II) mit dem Auge verträgliches Fluid, das nicht so eingeschlossen ist, welche Lösung umfaßt:

a) 0,0005 bis 0,1 Gew.-% eines Linsenabflachungsmittels, das ausgewählt wird aus der Gruppe Harnstoff, Glycerin, Sorbit, Aminoethanol und deren Gemische; b) ein für das Auge verträgliches ionisches Salz in einer Menge, die ausreichend ist, der gesamten Komfort-Tropflösung eine Tonizität im Bereich von 270 bis 330 milliosmol pro Kilogramm der genannten Komfort-Tropflösung zu verleihen; c) ein für das Auge verträgliches die Viskosität erhöhendes Mittel in ausreichender Menge, um der Komfort-Tropflösung eine relative Brookfield-Viskosität im Bereich von 3 bis 50 mPa.s (cps) zu verleihen; d) einen für das Auge verträglichen Puffer in einer Menge von 0 % oder einer wirksamen puffernden Menge, um zu einer Komfort-Tropflösung mit einem pH-Wert von 6,5 bis 8 zu führen; e) 0 % bis 0,5 % eines für das Auge verträglichen Konservierungsmittels; f) 0 % bis 2 % eines für das Auge verträglichen Gleitmittels; g) 0 % oder eine für das Auge annehmbare pharmazeutisch wirksame Menge eines okular verabreichbaren pharmazeutisch aktiven Mittels; und h) 0 % bis 1 % eines nichtionischen für das Auge annehmbaren oberflächenaktiven Mittels;
durch herkömmliches Mischen der Komponenten.

**22.** Verfahren nach Anspruch 21, worin das genannte Abflachungsmittel Harnstoff ist.

**23.** Verfahren nach Anspruch 21, worin das genannte Abflachungsmittel in einer Menge von 0,001 bis 0,08 Gew.-% vorliegt.

**24.** Verfahren nach Anspruch 21, worin das genannte Abflachungsmittel in einer Menge von 0,01 bis 0,05 Gew.-% vorliegt.

**25.** Verfahren nach Anspruch 21, worin die genannte relative Brookfield-Viskosität im Bereich von 3 bis 20 mPa.s (cps) liegt.

**26.** Verfahren nach Anspruch 25, worin die genannte relative Brookfield-Viskosität im Bereich von 6 bis 11 mPa.s (cps) liegt.

**27.** Verfahren nach Anspruch 21, worin das genannte die Viskosität erhöhende Mittel Hydroxyethylcellulose ist.

**28.** Verfahren nach Anspruch 21, worin das genannte ionische Salz in zumindest einer Menge vorhanden ist, die hinsichtlich der Osmolarität einer Lösung von 0,3 Gew.-% Natriumchlorid äquivalent ist.

**29.** Verfahren nach Anspruch 21, worin das genannte ionische Salz in zumindest einer Menge vorhanden ist, die hinsichtlich der Osmolarität einer Lösung von 0,5 Gew.-% Natriumchlorid äquivalent ist.

**30.** Verfahren nach Anspruch 21, worin das genannte ionische Salz in zumindest einer Menge vorhanden ist, die hinsichtlich der Osmolarität einer Lösung von 0,75 Gew.-% Natriumchlorid äquivalent ist.

**31.** Verfahren nach Anspruch 21, worin das genannte ionische Salz Natriumchlorid ist.

**32.** Verfahren nach Anspruch 21, worin das genannte Konservierungsmittel das Auge nicht irritiert.

**33.** Verfahren nach Anspruch 21, worin das genannte Konservierungsmittel in einer Menge von 0,001 bis 0,5 Gew.-% der Komfort-Tropfen vorliegt.

**34.** Verfahren nach Anspruch 21, worin das genannte Konservierungsmittel in einer Menge von 0,1 bis 0,25 Gew.-% der Komfort-Tropfen vorliegt.

**35.** Verfahren nach Anspruch 21, worin das genannte Konservierungsmittel Sorbinsäure ist.

**36.** Verfahren nach Anspruch 21, worin der pH-Wert der Komfort-Tropfen 7,0 bis 7,6 beträgt.

EP 0 198 490 B1

**37.** Verfahren nach Anspruch 21, worin der pH-Wert der Komfort-Tropfen 7,3 beträgt.

**38.** Verfahren nach Anspruch 21, worin der genannte Puffer ein Boratpuffer ist.

**39.** Verfahren nach Anspruch 21, worin das okular verabreichbare pharmazeutisch aktive Mittel ein Augen-Dekongestionsmittel in einer okular gefäßverengend wirksamen Menge vorhanden ist.

**40.** Verfahren nach Anspruch 21, worin die genannte Komfort-Tropflösung 0,03 Gew.-% Harnstoff; 0,52 Gew.-% NaCl; 0,16 Gew.-% Borsäure; 0,38 Gew.-% Na-Tetraborat-decahydrat; 0,2 Gew.-% Dinatrium-EDTA; 0,15 Gew.-% Sorbinsäure; 0,7 Gew.-% Hydroxyethylcellulose; 0,2 Gew.-% eines Ethylenoxid-Propylenoxid-Copolymers wie Poloxamer$^{(R)}$407; und Wasser umfaßt.

**41.** Verwendung einer Komfort-Tropflösung, umfassend
a) 0,1 bis 0,5 Gew.-% eines Linsenabflachungsmittels, das ausgewählt wird aus der Gruppe Harnstoff, Glycerin, Sorbit, Aminoethanol und deren Gemische; b) ein für das Auge verträgliches ionisches Salz in einer Menge, die ausreichend ist, der gesamten Komfort-Tropflösung eine Tonizität im Bereich von 270 bis 330 milliosmol pro Kilogramm der genannten Komfort-Tropflösung zu verleihen; c) ein für das Auge verträgliches die Viskostät erhöhendes Mittel in ausreichender Menge, um der Komfort-Tropflösung eine relative Brookfield-Viskosität im Bereich von 3 bis 50 mPa.s (cps) zu verleihen; d) einen für das Auge verträglichen Puffer in einer Menge von 0 % oder einer wirksamen puffernden Menge, um zu einer Komfort-Tropflösung mit einem pH-Wert von 6,5 bis 8 zu führen; e) 0 % bis 0,5 % eines für das Auge verträglichen Konservierungsmittels; f) 0 % bis 2 % eines für das Auge verträglichen Gleitmittels; g) 0 % eines okular verabreichbaren pharmazeutisch aktiven Mittels; und h) 0 % bis 1 % eines nichtionischen für das Auge annehmbaren oberflächenaktiven Mittels, zur Verbesserung des Austauschs von I) Fluid, das sich zwischen einer tagsüber zu tragenden Hydrogel-Kontaktlinse und einem Auge, auf das die Linse aufgebracht ist, befindet, gegen II) mit dem Auge verträgliches Fluid, das nicht so lokalisiert ist, während die Linse im Auge getragen wird, umfassend das Eintropfen einer den Fluidaustausch verbessernden wirksamen Menge der genannten Komfort-Tropflösung in das Auge, in dem sich die Linse befindet, wobei diese Linse temporär abgeflacht wird, was größeren Fluidaustausch ermöglicht als in Abwesenheit der genannten Komfort-Tropflösung möglich ist.

**42.** Verwendung nach Anspruch 41, worin die genannte Komfort-Tropflösung in einer Menge von 1 bis 5 Tropfen bis zu 5 Mal am Tag eingetropft wird.

**43.** Verwendung der Komfort-Tropflösung nach Anspruch 20 zur Verbesserung des Austauschs von I) Fluid, das sich zwischen einer tagsüber zu tragenden Hydrogel-Kontaktlinse und einem Auge, auf das die Linse aufgebracht ist, befindet, gegen II) mit dem Auge verträgliches Fluid, das nicht so lokalisiert ist, während die Linse im Auge getragen wird,
umfassend das Eintropfen einer den Fluidaustausch verbessernden wirksamen Menge der genannten Komfort-Tropflösung nach Anspruch 20 in das Auge, in dem sich die Linse befindet, wobei diese Linse temporär abgeflacht wird, was größeren Fluidaustausch ermöglicht als in Abwesenheit der genannten Komfort-Tropflösung nach Anspruch 20 möglich ist.

**44.** Verwendung nach Anspruch 43, worin die genannte Komfort-Tropflösung in einer Menge von 1 bis 5 Tropfen bis zu 5 Mal am Tag eingetropft wird.

**45.** Verwendung einer Komfort-Tropflösung nach Anspruch 21, worin die Komponente g) abwesend ist, zur Verbesserung des Austauschs von I) Fluid, das sich zwischen einer Hydrogel-Kontaktlinse für verlängerte Tragedauer und einem Auge, auf das die Linse aufgebracht ist, befindet, gegen II) mit dem Auge verträgliches Fluid, das nicht so lokalisiert ist, während die Linse im Auge getragen wird,
umfassend das Eintropfen einer den Fluidaustausch verbessernden wirksamen Menge der genannten Komfort-Tropflösung nach Anspruch 21 in das Auge, in dem sich die Linse befindet, wobei diese Linse temporär abgeflacht wird, was größeren Fluidaustausch ermöglicht als in Abwesenheit der genannten Komfort-Tropflösung nach Anspruch 21 möglich ist.

**46.** Verwendung nach Anspruch 45, worin die genannte Komfort-Tropflösung in einer Menge von 1 bis 5 Tropfen bis zu 5 Mal am Tag eingetropft wird.

34

**47.** Verwendung einer Komfort-Tropflösung nach Anspruch 40 zur Verbesserung des Austauschs von I) Fluid, das sich zwischen einer Hydrogel-Kontaktlinse für verlängerte Tragedauer und einem Auge, auf das die Linse aufgebracht ist, befindet, gegen II) mit dem Auge verträgliches Fluid, das nicht so lokalisiert ist, während die Linse im Auge getragen wird, umfassend das Eintropfen einer den Fluidaustausch verbessernden wirksamen Menge der genannten Komfort-Tropflösung nach Anspruch 40 in das Auge, in dem sich die Linse befindet, wobei diese Linse temporär abgeflacht wird, was größeren Fluidaustausch ermöglicht als in Abwesenheit der genannten Komfort-Tropflösung nach Anspruch 40 möglich ist.

**48.** Verwendung nach Anspruch 47, worin die genannte Komfort-Tropflösung in einer Menge von 1 bis 5 Tropfen bis zu 5 Mal am Tag eingetropft wird.